(19) Europäisches Patentamt
European Patent Office
Office européen des brevets

(11) EP 2 241 327 A1

(12) **EUROPEAN PATENT APPLICATION**

(43) Date of publication:
20.10.2010 Bulletin 2010/42

(51) Int Cl.:
*A61K 38/28* (2006.01)     *A61K 38/22* (2006.01)
*A61K 47/10* (2006.01)     *A61P 3/10* (2006.01)

(21) Application number: **10166765.7**

(22) Date of filing: **15.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **15.03.2006 EP 06111170**

(62) Document number(s) of the earlier application(s) in accordance with Art. 76 EPC:
**07726938.9 / 1 996 224**

(71) Applicant: **NOVO NORDISK A/S**
**2880 Bagsvaerd (DK)**

(72) Inventors:
• **Schlein, Morten**
  **DK-2300 Copenhagen (DK)**
• **Hansen, Thomas Kruse**
  **DK-2730 Herlev (DK)**
• **Lau, Jesper**
  **3520 Farum (DK)**
• **Ludvigsen, Svend**
  **3540 Lynge (DK)**

Remarks:
This application was filed on 22-06-2010 as a divisional application to the application mentioned under INID code 62.

(54) **Mixtures of Amylin and Insulin**

(57)     The present invention relates to a soluble pharmaceutical composition for parenteral administration, which comprises an amylin peptide, and a meal-related insulin peptide, and to the use of such compositions for treatment of e.g. hyperglycemia.

EP 2 241 327 A1

**Description**

**FIELD OF THE INVENTION**

The present invention relates to the field of pharmaceutical compositions. More specifically the invention pertains to pharmaceutical compositions comprising two different pharmaceutically active peptides.

**BACKGROUND OF THE INVENTION**

Diabetes mellitus is a metabolic disorder in which the ability to utilize glucose is partly or completely lost. About 5% of all people suffer from diabetes and the disorder approaches epidemic proportions. Since the introduction of insulin in the 1920's, continuous efforts have been made to improve the treatment of diabetes mellitus. Since people suffering from diabetes are subject to chronic treatment over several decades, there is a major need for safe, convenient and life quality improving insulin formulations.

In the treatment of diabetes mellitus, many varieties of insulin formulations have been suggested and used, such as regular insulin, isophane insulin (designated NPH), insulin zinc suspensions (such as Semilente®, Lente®, and Ultralente®), and biphasic isophane insulin. Some of the commercial available insulin formulations are characterized by a fast onset of action and other formulations have a relatively slow onset but show a more or less prolonged action. Fast-acting insulin formulations are usually solutions of insulin, while retarded acting insulin formulations can be suspensions containing insulin in crystalline and/or amorphous form precipitated by addition of zinc salts alone or by addition of protamine or by a combination of both.

Normally, insulin formulations are administered by subcutaneous injection. What is important for the patient is the action profile of the insulin formulation which is the action of insulin on the glucose metabolism as a function of the time from the injection. In this profile, inter alia, the time for the onset, the maximum value, and the total duration of action are important. A variety of insulin formulations with different action profiles are desired and requested by the patients.

Human insulin consists of two polypeptide chains, the so-called A and B chains which contain 21 and 30 amino acid residues, respectively. The A and B chains are interconnected by two cystine disulphide bridges. Insulin from most other species has a similar construction, but may not contain the same amino acid residues at the same positions. Within the last decade a number of human insulin analogs have been developed. They are designed for particular profiles of action, i.e. fast acting or prolonged action.

Another peptide of interest in the treatment of diabetes is amylin. Human amylin is a 37 amino acid long peptide which has physico-chemical properties that make its use as a drug troublesome. In particular, it has a tendency to fibrillate ex-vivo and become ineffective due to precipitation. There is currently on the marked a drug product called Symlin containing an analog of human amylin (pramlintide) where the three amino acids in positions 25, 28 and 29 each are substituted to proline. This improves substantially the fibrillating tendency. However, even pramlintide is difficult to keep in solution at neutral pH and it is therefore provided in an acidic solution i.e. Symlin.

The actions of amylin in relation to diabetes are: Reduction of food-intake leading to lower body-weight, slower gastric emptying, smoothening of post-prandial glucose profiles, and reduction in the excessive diabetic glucagon release (A. Young, Amylin: Physiology and Pharmacology, Academic Press (2005)). By and large the actions of amylin are mediated via identified CNS receptors rather than directly on the target organs.

Symlin is approved as an adjunct drug with insulin. Clinical trials have revealed improved HbAlc in the order of 0.3-0.6, a smoother and shallower post-prandial blood glucose profile and reduction in body weight in contrast to treatment with insulin alone. Symlin is currently administered as a separate injection at a separate injection site three times daily. If the patient also uses three insulin injections per day this adds to a total of six daily injections.

Symlin therapy is limited by nausea as a side-effect. The nausea is dose-related but has a tendency to diminish with time. The pharmaco-kinetic profile of Symlin leads to rather large variations in plasma levels throughout the day. It takes approximately 20 minutes after a subcutaneous injection for Symlin to reach $C_{max}$ and plasma t½ is in the order of 20 minutes. Ultimately this leads to a need for three or more daily injections of Symlin in order to keep pharmacological plasma level without substantial side-effects. Even with three daily injections Symlin does not mimic the natural release profile of amylin very well. Amylin is released as meal related peaks with a duration close to 3-6 hours in contrast to the 1-1½ hour duration of an injected Symlin profile. Amylin also has a substantial basal level that is not mimicked by Symlin (A. Young, Amylin: Physiology and Pharmacology, Academic Press (2005)).

It would be useful to provide a pharmaceutical composition combining an amylin peptide, and a meal-related insulin peptide in a stable solution in order to be able to better mimic the physiological profile of the peptides in a patient in response to glucose metabolism and limit the number of daily injections.

**BRIEF DESCRIPTION OF THE DRAWINGS**

Figure 1 illustrates the solubility of a mixture of the amylin analogue pramlintide ($^{25,28,29}$Pro- h-amylin) and insulin$^{A21G, B28D, desB30}$ versus pH.

Figure 2 illustrates the physical stability of a mixture containing insulin$^{A21G, B28D, desB30}$ and pramlintide ($^{25,28,29}$ Pro- h-amylin) using a ThT fibrillation assay.

Figure 3 illustrates the solubility of a mixture of the amylin analogue pramlintide ($^{25,28,29}$Pro- h-amylin) and insulin$^{A21G, B28E, desB30}$ versus pH.

Figure 4 illustrates the physical stability of a mixture of insulin$^{A21G, B28E, desB30}$ and the amylin analogue pramlintide ($^{25,28,29}$ Pro- h-amylin) using a ThT fibrillation assay.

Figure 5 illustrates the solubility of a mixture of 0.6 mM insulin$^{B28D}$, 0.2 mM $Zn(Ac)_2$, 0.15 mM pramlintide ($^{25,28,29}$ Pro- h-amylin) versus pH.

Figure 6 illustrates the solubility of a mixture of 0.6mM Insulin$^{B211D}$, 0.2 mM $Zn(Ac)_2$, 0.1 mM pramlintide ($^{25,28,29}$Pro- h-amylin), 16mM phenol, 16mM m-cresol, 3000 ppm Poloxamer-188.

Figure 7 illustrates the effect of a phospholipid on the physical stability of a mixture containing both insulin$^{B28D}$ and pramlintide ($^{25,28,29}$Pro- h-amylin).

Figure 8 illustrates the solubility of a mixture of 0.6 mM insulin$^{B211D}$, 0.2 mM $Zn(Ac)_2$, 100 $\mu$M pramlintide ($^{25,28,29}$Pro- h-amylin), 30 mM phenol, and 3 mM 1,2-Dimyristoyl-sn-glycero-3-phospho-rac-(1-glycerol) (DMPG).

Figure 9 illustrates the physical stability of mixtures containing insulin$^{B28D}$, pramlintide ($^{25,28,29}$Pro-h-amylin), and DMPG using a ThT fibrillation assay.

Figure 10 illustrates that the time course for fibril formation can be described by a sigmoidal curve.

**SUMMARY OF THE INVENTION**

One object of the present invention is to provide a soluble pharmaceutical composition for parenteral administration, which comprises an amylin peptide, and a meal-related insulin peptide.

A further object of the invention is to provide a method for treatment of hyperglycemia comprising parenteral administration of an effective amount of a soluble pharmaceutical composition for parenteral administration, which comprises an amylin peptide, and a meal-related insulin peptide.

**DEFINITIONS**

The following is a detailed definition of some of the terms used in the specification.

The term "effective amount" as used herein means a dosage which is sufficient in order for the treatment of the patient to be effective compared with no treatment.

The term "medicament" as used herein means a pharmaceutical composition suitable for administration of the pharmaceutically active compounds to a patient.

The term "pharmaceutical composition" as used herein means a product comprising an active compound or a salt thereof optionally together with pharmaceutical excipients such as a buffer, preservative and tonicity modifier, said pharmaceutical composition being useful for treating, preventing or reducing the severity of a disease or disorder by administration of said pharmaceutical composition to a person. Thus, a pharmaceutical composition is also known in the art as a pharmaceutical formulation.

The term "soluble pharmaceutical composition" as used herein means an amylin peptide which is substantially soluble, and a meal-related insulin peptide which is substantially soluble in the combined composition. Thus, a predissolved soluble pharmaceutical composition will be substantially soluble, and a soluble pharmaceutical composition which is to be reconstituted will be substantially soluble once it has been dissolved in the prescribed reconstitution liquid. It is to be understood that pH of a pharmaceutical composition which is to be reconstituted is the pH value which is measured on the reconstituted composition produced by reconstitution in the prescribed reconstitution liquid at room temperature.

The term "pharmaceutically acceptable" as used herein means suited for normal pharmaceutical applications, i.e. giving rise to no serious side effects such as adverse events in patients etc.

The term "buffer" as used herein refers to a chemical compound in a pharmaceutical composition that reduces the tendency of pH of the composition to change over time as would otherwise occur due to chemical reactions. Buffers include chemicals such as sodium phosphate, TRIS, glycine and sodium citrate.

The term "preservative" as used herein refers to a chemical compound which is added to a pharmaceutical composition to prevent or delay microbial activity (growth and metabolism). Examples of pharmaceutically acceptable preservatives are phenol, m-cresol and a mixture of phenol and m-cresol.

The term "isotonicity agent" as used herein refers to a chemical compound in a pharmaceutical composition that serves to modify the osmotic pressure of the pharmaceutical composition so that the osmotic pressure becomes closer to that of human plasma. Isotonicity agents include NaCl, glycerol, mannitol etc.

The term "stabiliser" as used herein refers to chemicals added to peptide containing pharmaceutical compositions in order to stabilize the peptide(s), i.e. to increase the shelf life and/or in-use time of such compositions. Examples of stabilisers used in pharmaceutical formulations are L-glycine, L-histidine, arginine, polyethylene glycol, and carboxymethylcellulose.

The term "surfactant" as used herein refers to any molecules or ions that are comprised of a water-soluble (hydrophilic) part, the head, and a fat-soluble (lipophilic) segment (the tail) as e.g. described in "Surfactants and Polymers in Aqueous Solution", 2nd ed. by K. Holmberg, B. Lindman et. al.. Surfactants accumulate preferably at interfaces, which the hydrophilic part is orientated towards the water (hydrophilic phase) and the lipophilic part towards the oil- or hydrophobic phase (*i.e.* glass, air, oil etc.). The concentration at which surfactants begin to form micelles is known as the critical micelle concentration or CMC. Furthermore, surfactants lower the surface tension of a liquid. Surfactants are also known as amphipathic compounds. The term "Detergent" is a synonym used for surfactants in general.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "analog" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been added to the peptide. In one aspect of the invention, such addition or deletion of amino acid residues can take place at the N-terminal of the peptide and/or at the C-terminal of the peptide. In one embodiment an analog comprises less than 8 modifications (substitutions, deletions, additions) relative to the native peptide. In one embodiment an analog comprises less than 7 modifications (substitutions, deletions, additions) relative to the native peptide. In one embodiment an analog comprises less than 6 modifications (substitutions, deletions, additions) relative to the native peptide. In another embodiment an analog comprises less than 5 modifications (substitutions, deletions, additions) relative to the native peptide. In another embodiment an analog comprises less than 4 modifications (substitutions, deletions, additions) relative to the native peptide. In another embodiment an analog comprises less than 3 modifications (substitutions, deletions, additions) relative to the native peptide. In another embodiment an analog comprises less than 2 modifications (substitutions, deletions, additions) relative to the native peptide. In another embodiment an analog comprises only a single modification (substitutions, deletions, additions) relative to the native peptide.

In one embodiment, the modification(s) are substitution(s). In another embodiment, the modification(s) are deletions(s). In another embodiment, the modification(s) are addition(s).

The term "derivative" as used herein in relation to a peptide means a chemically modified parent protein or an analog thereof, wherein at least one substituent is not present in the parent protein or an analog thereof, i.e. a parent protein which has been covalently modified. Typical modifications are amides, carbohydrates, alkyl groups, acyl groups, esters, PEGylations and the like. Examples of derivatives of human insulin are threonine methyl ester[B30] human insulin and N[εB29]-tetradecanoyl des(B30) human insulin.

The term "insulin peptide" as used herein means a peptide which is either human insulin or a chemically modified human insulin, such as an analog or a derivative thereof.

The term "human insulin" as used herein means the human hormone whose structure and properties are well-known. Human insulin has two polypeptide chains that are connected by disulphide bridges between cysteine residues, namely the A-chain and the B-chain. The A-chain is a 21 amino acid peptide and the B-chain is a 30 amino acid peptide, the two chains being connected by three disulphide bridges: one between the cysteines in position 6 and 11 of the A-chain, the second between the cysteine in position 7 of the A-chain and the cysteine in position 7 of the B-chain, and the third between the cysteine in position 20 of the A-chain and the cysteine in position 19 of the B-chain.

Mutations in the insulin molecule is denoted in superscript stating the chain (A or B), the position, and the single letter code for the amino acid substituting the native amino acid. By "desB30" is meant a natural insulin B chain or an analogue thereof lacking the B30 amino acid. Thus, human insulin[A21G, B28D, desB30] is an analogue of human insulin where position 21 in the A chain is mutated to glycine, position 28 in the B chain is mutated to aspartic acid, and position 30 in the B chain is deleted.

The term "meal-related insulin peptide" as used herein means an insulin peptide which has a time-action of less than 8 hours in standard models of diabetes. e.g. pharmacokinetic disappearance and/or appearance" in pigs.

Preferably, the meal-related human insulin has a time-action of less than about 5 hours. Preferably, the meal-related insulin has a time-action in the range from 0 hours to about 4 hours. Preferably, the meal-related insulin has a time-action similar to that observed for commercial pharmaceutical compositions of Actrapid®, Novolog®, Humalog® and Apidra®. The term about in relation to the time-action of insulins means + or - 30 minutes

The term "amylin peptide" as used herein means a peptide which is amylin, an analog or derivative thereof or an amylin agonist. It is understood that biological active amylin agonists may have an amide group attached to the acid group of the C terminal residue via a peptide bond.

Analogs of human amylin (h-amylin) may be denoted by the changed position or positions in superscript followed by the three letter code for the amino acid substituting the native amino acid or amino acids if several residues are changed to the same amino acid. Thus, pramlintide may be denoted as [25, 28, 29]Pro-h-amylin indicating that the positions 25, 28 and 29 in human amylin all have been changed to proline. By "des-[1]Lys" is meant that the lysine in position 1 is lacking, e.g. des-[1]Lys[18]Arg[25,28]Pro-h-amylin is a human amylin analog where the lysine in position 1 is lacking, the amino acid in position 18 is changed to an arginine, and the two amino acids in positions 25 and 28 are each changed to a proline.

The term "isoelectric point" as used herein means the pH value where the overall net charge of a macromolecule such as a peptide is zero. In peptides there may be several charged groups, and at the isoelectric point the sum of all these charges is zero. At a pH above the isoelectric point the overall net charge of the peptide will be negative, whereas at pH values below the isoelectric point the overall net charge of the peptide will be positive.

The term "reconstituted" as used herein referring to a pharmaceutical composition means an aqueous composition which has been formed by the addition of water to a solid material comprising the active pharmaceutical ingredient. Pharmaceutical compositions for reconstitution are applied where a liquid composition with acceptable shelf-life cannot be produced. An example of a reconstituted pharmaceutical composition is the solution which results when adding water to a freeze dried composition. The solution is often for parenteral administration and thus water for injection is typically used for reconstituting the solid material.

The term "about" as used herein in relation to the concentration of a peptide in a pharmaceutical composition means plus or minus 10%. Hence, the concentration "about 5 mg/mL insulin" means a concentration of 4.5 mg/mL insulin to 5.5 mg/mL insulin. In one embodiment, where the term "about" is used the corresponding value or range includes the exact value or range as if the term was not present.

## DESCRIPTION OF THE INVENTION

In one aspect, the invention relates to a soluble pharmaceutical composition for parenteral administration, which comprises an amylin peptide, and a meal-related insulin peptide.

In a further aspect of the invention, the pH of said pharmaceutical composition or a reconstituted solution of said pharmaceutical composition is from about pH 6.5 to about pH 9.0. In a further aspect of the invention, the pH of said pharmaceutical composition or a reconstituted solution of said pharmaceutical composition is from about pH 6.8 to about pH 8.0. In a further aspect of the invention, the pH of said pharmaceutical composition or a reconstituted solution of said pharmaceutical composition is from about pH 7.0 to about pH 7.8. In a further aspect of the invention, the pH of said pharmaceutical composition or a reconstituted solution of said pharmaceutical composition is from about pH 7.2 to about pH 7.6.

In another aspect of the invention, the pharmaceutical composition is a solution. In another aspect of the invention, the pharmaceutical composition is a solid. In another aspect of the invention, the pharmaceutical composition is to be reconstituted with an aqueous solution, such as a buffer or water for injection. In another aspect of the invention, the pharmaceutical composition is suitable for administration by injection or infusion. In a further aspect of the invention, the pharmaceutical composition is suitable for administration for subcutaneous administration. In another aspect of the invention, the pharmaceutical composition is suitable for intramuscular administration. In another aspect of the invention, the pharmaceutical composition is suitable for intravenous administration.

A further embodiment of the present invention relates to a pharmaceutical composition wherein the meal-related insulin peptide has a time action of less than 4 hours.

In another aspect, the present invention relates to a pharmaceutical composition wherein said meal-related insulin peptide is human insulin, an analog of human insulin, a derivative of human insulin or a derivative of a human insulin analog.

In one embodiment of the invention, said meal-related insulin peptide is human insulin. In a further embodiment of the invention, said meal-related insulin peptide is a human insulin analog. In a further embodiment of the invention, said meal-related insulin peptide is a derivative of a human insulin analog.

In one embodiment of the invention, the pH of the said pharmaceutical composition or a reconstituted solution of said pharmaceutical composition is from about pH 2 to about pH 4.5. In a further embodiment of the invention, the pH of said pharmaceutical composition or a reconstituted solution of said pharmaceutical composition is from about pH 2.5 to about pH 4.0. In a further embodiment of the invention, the pH of said pharmaceutical composition or a reconstituted solution of said pharmaceutical composition is from about pH 3.0 to about pH 4.0.

In another embodiment, the insulin analogs and derivatives are selected from among those disclosed in EP 0 792 290 (Novo Nordisk A/S), EP 0 214 826 and EP 0 705 275 (Novo Nordisk A/S), US 5,504,188 (Eli Lilly), EP 0 368 187 (Aventis), US patents 5,750,497 and 6,011,007, EP 375437 and EP 383472 and where such insulins may include, but are not limited to, insulin glulisine (also known as Apidra®, differs from human insulin in that the amino acid asparagine at position B3 is replaced by lysine and the lysine in position B29 is replaced by glutamic acid), human insulin$^{B28K, B29P}$ (Humalog®), and human insulin$^{B28D}$ (insulin aspart (Novolog®)).

In another aspect of the invention, the insulin analogs are selected from among those acid stabilised insulin analogs disclosed in WO2004/080480 such as the human insulin analog human insulin$^{A21G, B28D, des B30}$. In one embodiment of the invention, the meal-related insulin peptide is human insulin$^{A21G, B28D, des B30}$. In another embodiment of the invention, the meal-related insulin peptide is human insulin$^{A21G, B28E, des B30}$. The mutation A21G in insulin analogues such as insulin$^{A21G, B28D, des B30}$ confers chemical stability of the insulin analogue at acidic pH, since the native asparagine at this position is susceptible for deamidation at acidic pH. The B28D mutation renders the insulin monomeric and hence useful as a meal-related insulin. In one aspect of the invention, the meal-related insulin peptide comprises mutations, which renders the insulin peptide stabilised against acidic deamidation. In one aspect of the invention, the insulin peptide comprises mutation of A21Asn to Ala, Val, Leu, Ile, Pro, Phe, Trp, Gly, Ser, Thr, Tyr, Asp, Glu, Lys, Arg, or His. In another aspect of the invention, the meal-related insulin peptide comprises mutation of A21Asn to Gly or Ala. In a further aspect of the invention, the meal-related insulin peptide comprises mutation of A21Asn to Gly. In yet a further aspect of the invention, the meal-related insulin peptide comprises at least one amino acid attached to A21Asn via a peptide bond. In yet a further aspect of the invention, the meal-related insulin peptide comprises two amino acids attached to A21Asn via a peptide bond. In another aspect of the invention, the meal-related insulin peptide comprises one amino acid attached to A21Asn via a peptide bond.

In the embodiment of the invention, where the meal-related insulin peptide is an acid stabilised insulin analog, the pH of a pharmaceutical composition or a reconstituted solution of said pharmaceutical composition comprising an acid stabilised insulin analog may in a preferred embodiment be from about pH 2 to about pH 4.5. In a further embodiment of the invention, the pH of said pharmaceutical composition or a reconstituted solution of said pharmaceutical composition is from about pH 2.5 to about pH 4.0. In a further embodiment of the invention, the pH of said pharmaceutical composition or a reconstituted solution of said pharmaceutical composition is from about pH 3.0 to about pH 4.0.

In one embodiment of the invention, said human insulin analog is human insulin$^{B28D}$ (also known as Asp$^{B28}$-human insulin). In another embodiment of the invention, said human insulin analog is human insulin$^{B28K, B29P}$ (also known as Lys$^{B28}$, Pro$^{B29}$-human insulin). In another embodiment of the invention, said human insulin analog is human insulin$^{B3K,B29E}$ (also known as Lys$^{B3}$,Glu$^{B29}$-human insulin) (insulin glulisine). In another embodiment of the invention, said human insulin analog is human insulin$^{desB30}$ (also known as des(B30) human insulin)

In another embodiment of the invention, the concentration of said meal-related insulin peptide in said pharmaceutical composition is in the range from about 1.6 mg/mL to about 5.6 mg/mL, or from about 2.6 mg/mL to about 4.6 mg/mL, or from about 3.2 mg/mL to about 4.0 mg/mL. In another embodiment of the invention, the concentration of said meal-related insulin peptide is in the range from about 1.2 mg/mL to about 5.6 mg/mL.

In another embodiment of the invention, the concentration of said meal-related insulin peptide in said pharmaceutical composition is in the range from about 1 mg/mL to about 10 mg/mL, or from about 2.5 mg/mL to about 8.75 mg/mL, or from about 3.5 mg/mL to about 8.75 mg/mL, or from about 5 mg/mL to about 8.75 mg/mL.

In another embodiment of the invention, the pharmaceutical composition comprises two different insulin peptides.

In one aspect of the invention, the amylin peptide is amylin, an amylin analog or an amylin agonist.

"Amylin" as used herein refers to compounds such as those described in U. S. Patents 5,124,314 and 5,234,906, both of which are hereby incorporated by reference. The term includes, but is not limited to, a human peptide hormone of 37 amino acids referred to as amylin, which is co-secreted with insulin from β-cells of the pancreas. Human amylin has the following amino acid sequence: Lys-Cys-Asn-Thr-Ala-Thr-Cys-Ala-Thr-Gln-Arg-Leu-Ala- Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr (SEQ ID NO:1). Thus, the structural formula is Lys-Cys-Asn-Thr-Ala-Thr-Cys-Ala-Thr-Gln-Arg-Leu-Ala- Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr-NH$_2$ (SEQ ID NO: 1) with a disulfidebridge between the two Cys residues and an amide group attached to the C-terminal amino acid via a peptide bond. The term also includes variants of amylin as present in, and in isolatable form, other mammalian species. With respect to a naturally occurring amylin compound, the term includes such a compound in an isolated, purified, or other form that is otherwise not found in nature.

An "agonist" of amylin refers to a compound that mimics one or more effects (or activity) of amylin *in vitro* or *in vivo.* The effects of amylin include the ability to directly or indirectly interact or bind with one or more receptors that are activated or deactivated by amylin, for example, the receptor binding assay and the soleus muscle assay described in Examples 2 and 3, respectively in WO 2004/037168. Preferably, the amylin agonist is not a calcitonin, which, as used herein, refers to the human peptide hormone calcitonin and species variations of it, such as that of rat, salmon 10 and eel (including aminosuberic eel calcitonin).

An "analog" (or "analogue" or "agonist analog") of amylin refers to a compound that is similar in structure (e.g., derived from the primary amino acid sequence of amylin by substituting one or more natural or unnatural amino acids or peptidomimetics) to amylin and mimics an effect of amylin *in vitro* or *in vivo.* Amylin analogs useful according to the invention may also include fragments of amylin such as those described in EP 289287, the contents of which are herein incorporated by reference. Preferred amylin agonists may also be compounds having at least 60, 65, 70, 75, 80, 85, 90, 95, or 99% amino acid sequence identity to SEQ ID NO: 1 and having amylin activity. The amylin peptide of the present invention may be capable of binding to or otherwise directly or indirectly interacting with an amylin receptor, or other receptor or receptors with which amylin itself may interact to elicit a biological response, e.g., reducing food intake. Compounds of the invention may bind an amylin receptor with an affinity of greater than 20 nM, 10 nM, 5 nM, and more preferably with an affinity of greater than 0.10 nM e.g. as determined by the amylin receptor assay in the section "Methods".

Amylin analogs also include amylin having insertions, deletions, and/or substitutions in at least one or more amino acid positions of SEQ ID NO: 1. The number of amino acid insertions, deletions, or substitutions may be at least 1, 2, 3, 4, 5, 6, 10, 15, 20, 25, or 30. Insertions or substitutions may be with other natural or unnatural amino acids, synthetic amino acids, peptidomimetics, or other chemical compounds.

A "derivative" of amylin refers to an amylin which is chemically modified such as N-methylated amylin. In one aspect of the invention, the amylin peptide is amylin N-methylated in positions 24 and 26 as described in Yan et al, PNAS, vol. 103, no. 7, p. 2046-2051, 2006.

Exemplary amylin agonist analogs contemplated in the use of the invention include those described in U.S. Patent Nos. 5,686,411, 6,114, 304, and 6,410,511, which are herein incorporated by reference in their entirety.

Exemplary compounds include, but are not limited to des-[1]Lys-h-amylin, [28]Pro-h-amylin, [25, 28, 29]Pro-h-amylin, [18]Arg[25, 28]Pro-h-amylin, and des-[1]Lys[18]Arg[25, 28]Pro-h-amylin, all show amylin activity *in vivo* in treated test animals, (e.g. provoking marked hyperlactemia followed by hyperglycemia). In addition to having activities characteristic of amylin, certain of the preferred compounds of the invention have also been found to possess more desirable solubility and stability characteristics when compared to human amylin. Examples of these compounds include [25]Pro[26]Val[28, 29]Pro-h-amylin,[25, 28, 29]Pro-h-amylin, and [18]Arg[25, 28]Pro-h- amylin.

In one aspect of the invention, said amylin peptide is human amylin. In one aspect of the invention, said amylin peptide is [25 ,28 ,29]Pro- h-amylin (pramlintide). In a further aspect of the invention, said amylin peptide is human amylin methylated in position 24 and 26.

In one aspect of the invention, the concentration of said amylin peptide is in the range from about 0.05 mg/mL to about 10 mg/mL or from about 0.1 mg/mL to about 4 mg/mL, or from about 0.4 mg/mL to about 1.2 mg/mL.

In one aspect of the invention, said meal-related insulin peptide is human insulin and said amylin peptide is [25, 28, 29]Pro- h-amylin. In a further aspect of the invention, the concentration of said amylin peptide is in the range from about 0.05 mg/mL to about 10 mg/mL and the concentration of human insulin is in the range from about 3.2 mg/mL to about 4.0 mg/mL.

In another aspect of the invention, said insulin peptide is human insulin[B28D] and said amylin peptide is [25, 28, 29]Pro- h-amylin. In a further aspect of the invention, the concentration of [25 ,28 29]Pro- h-amylin is in the range from about 0.05 mg/mL to about 10 mg/mL and the concentration of human insulin[B28D] is in the range from about 0.3 mg/mL to about 4.0 mg/mL. In yet a further aspect of the invention, the concentration of [25, 28 ,29]Pro- h-amylin is in the range from about 0.1 mg/mL to about 4 mg/mL, and the concentration of human insulin[B28D] is in the range from about 0.36 mg/mL to about 3.8 mg/mL.

In another aspect of the invention, said insulin peptide is human insulin[B3K, B29E] and said amylin peptide is [25, 28, 29]Pro- h-amylin. In a further aspect of the invention, the concentration of human insulin[B3K, B29E] is in the range from about 0.36 mg/mL to about 4.0 mg/mL.

In another aspect of the invention, said insulin peptide is human insulin[B28K, B29P] and said amylin peptide is [25,28,29]Pro- h-amylin. In a further aspect of the invention, the concentration of human insulin[B28K, B29P] is in the range from about 0.36 mg/mL to about 4.0 mg/mL.

In one aspect, the present invention relates to a pharmaceutical composition which further zinc and/or calcium. In one aspect, the present invention relates to a pharmaceutical composition which further comprises zinc. In another aspect, the present invention relates to a pharmaceutical composition which further comprises calcium. In one embodiment of the invention, the molar ratio of zinc to insulin peptide is from 1/6 to ½ mole/mole, preferable from 3/12 to 5/12 mole/mole. In a further embodiment of the invention, the molar ratio of calcium to insulin peptide is from 1/12 to 5/12 mole/mole, preferable from 1/6 to 1/3 mole/mole, such as from 3/12 to 5/12 mole/mole.

The pharmaceutical compositions of the invention are chemically stable and soluble at the desired pH. By "soluble at a given pH" is meant that the insulin peptide and/or the amylin peptide contained in the composition of the invention is fully dissolved at the pH of the composition where methods for determining whether the insulin peptide and/or the amylin peptide contained in the composition of the invention are dissolved are known in the art.

In one embodiment, the pharmaceutical composition may be subjected to centrifugation for 20 minutes at 30,000 g and then insulin peptide and/or the amylin peptide concentration in the supernatant may be determined by RP-HPLC. If this concentration is equal within experimental error to the insulin peptide and/or the amylin peptide concentration originally used to make the composition, then the insulin peptide and/or the amylin peptide is fully soluble in the composition of the invention.

In another embodiment, the solubility of the insulin and/or the amylin peptide(s) in a composition of the invention can simply be determined by examining by eye the container in which the composition is contained. The insulin and/or the amylin peptide(s) is soluble if the solution is clear to the eye and no particulate matter is either suspended or precipitated on the sides/bottom of the container.

In another embodiment, the physical stability of the insulin and/or the amylin peptide(s) in a composition of the invention can be determined by a ThT fibrillation assay for the assessment of physical stability of protein formulations.

In one embodiment of the invention, the pharmaceutical composition according to the invention is a "physical stable" pharmaceutically composition. The term "physical stable" as used in this context means that the amylin peptide and the meal-related insulin peptide does not destabilize each other in the combined composition i.e. the pharmaceutical composition is as physical stable as the least stable of the amylin peptide and the meal-related insulin peptide alone. Physical stability may be determined as described in the ThT fibrillation assay under "Methods".

Of course, it is to be understood by the skilled artisan that the solubility of the insulin and/or the amylin peptide(s) in a composition of the invention may be affected not only by the composition and its pH but also by the temperature and time at which the composition is stored prior to measurement of solubility. For example, while storage of the composition of the invention at a temperature of 45°C may narrow the range of pH values where solubility is observed, any precipitation of the insulin and/or the amylin peptide(s) from the compositions of the invention observed at 45°C may be reversed by lowering the temperature of the composition. Thus, for example, if a composition is a clear solution (i.e. soluble) at 4°C at a given pH between pH 5-6 but starts to precipitate when stored at 45°C, this precipitate will be resolvated when the composition is stored at 4°C afterwards.

In one embodiment of the invention, said pharmaceutical composition comprises a preservative. In one embodiment said preservative is selected from the group consisting phenol, m-cresol or a mixture thereof.

In a further embodiment of the invention, said pharmaceutical composition comprises a buffer. In one embodiment said buffer is selected from the group consisting phosphate, a

Good's buffer such as TRIS, BICINE, and HEPES, glycine, glycylglycine, citrate or a mixture thereof.

In a further embodiment of the invention, said pharmaceutical composition comprises an isotonicity agent. In one embodiment, said isotonicity agent is not a salt. In a further embodiment, said isotonicity agent is selected from the group consisting of trehalose, glucose, mannitol, sorbitol, glycerol, propylene glycol and a mixture thereof.

In a further embodiment of the invention, said pharmaceutical composition comprises a stabiliser. In one embodiment, said stabiliser is selected from the group consisting of L-histidine, imidazole and L-arginine. In one embodiment, said stabiliser is a polyethylene glycol.

In a further embodiment of the invention, said pharmaceutical composition comprises a surfactant. As examples of surfactants mention can be made of anionic surfactants, cationic surfactants, nonionic surfactants, and zwitterionic surfactants.

In one aspect of the invention, the surfactant is an anionic surfactant.

In one aspect of the invention, the pharmaceutical composition comprises an anionic surfactant in a concentration higher than its critical micelle concentration (CMC).

Anionic surfactants may be selected from the group of: Chenodeoxycholic acid, Chenodeoxycholic acid sodium salt, Cholic acid, Dehydrocholic acid, Deoxycholic acid, Deoxycholic acid methyl ester, Digitonin, Digitoxigenin, N,N-Dimethyldodecylamine N-oxide, Docusate sodium, Glycochenodeoxycholic acid sodium, Glycocholic acid hydrate, Glycodeoxycholic acid monohydrate, Glycodeoxycholic acid sodium salt, Glycodeoxycholic acid sodium salt, Glycolithocholic acid 3-sulfate disodium salt, Glycolithocholic acid ethyl ester, N-Lauroylsarcosine sodium salt, N-Lauroylsarcosine sodium salt, N-Lauroylsarcosine, N-Lauroylsarcosine, Lithium dodecyl sulfate, Lugol, 1-Octanesulfonic acid sodium salt, 1-Octanesulfonic acid sodium salt, Sodium 1-butanesulfonate, Sodium 1-decanesulfonate, Sodium 1-dodecanesulfonate, Sodium 1-heptanesulfonate, Sodium 1-heptanesulfonate, Sodium 1-nonanesulfonate, Sodium 1-propanesulfonate monohydrate, Sodium 2-bromoethanesulfonate, Sodium cholate hydrate, ox or sheep bile, Sodium cholate hydrate, Sodium choleate, Sodium deoxycholate, Sodium dodecyl sulfate, Sodium dodecyl sulfate, Sodium hexanesulfonate, Sodium octyl sulfate, Sodium pentanesulfonate, Sodium taurocholate, Taurochenodeoxycholic acid sodium salt, Taurodeoxycholic acid sodium salt monohydrate, Taurolithocholic acid 3-sulfate disodium salt, Tauroursodeoxycholic acid sodium salt, Trizma® dodecyl sulfate, DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), Dodecylphosphocholine (FOS-Choline-12), Decylphosphocholine (FOS-Choline-10), Nonylphosphocholine (FOS-Choline-9), dipalmitoyl phosphatidic acid, sodium caprylate, and/or Ursodeoxycholic acid.

In one aspect of the invention, the anionic surfactant is a glycerophosphoglycerol derivative. In one aspect of the invention, said glycerophosphoglycerol derivative is a dimyristoyl derivative, e.g. 1,2-Dimyristoyl-*sn*-glycero-3-phospho-rac-(1-glycerol). In one aspect of the invention, the dimyristoyl derivative is added in a concentration between 0.1 mM and 10 mM, preferably between 0.5 mM and 5 mM, preferably between 0.5 mM and 3 mM. In one aspect of the invention, said glycerophosphoglycerol derivative is 1,2-Dimyristoyl-sn-glycero-3-phospho-rac-(1-glycerol) (DMPG), such as CAS no. 67232-80-8.

In one aspect of the invention, the surfactant is a cationic surfactant.

Cationic surfactants may be selected from the group of: Alkyltrimethylammonium bromide, Benzalkonium chloride, Benzalkonium chloride, Benzyldimethylhexadecylammonium chloride, Benzyldimethyltetradecylammonium chloride, Benzyltrimethylammonium tetrachloroiodate, Dimethyldioctadecylammonium bromide, Dodecylethyldimethylammonium bromide, Dodecyltrimethylammonium bromide, Dodecyltrimethylammonium bromide, Ethylhexadecyldimethylammonium bromide, Hexadecyltrimethylammonium bromide, Hexadecyltrimethylammonium bromide, Polyoxyethylene (10)-N-tallow-1,3-diaminopropane, Thonzonium bromide, and/or Trimethyl(tetradecyl)ammonium bromide.

In one aspect of the invention, the surfactant is a nonionic surfactant.

Nonionic surfactants may be selected from the group of: BigCHAP, Bis(polyethylene glycol bis[imidazoyl carbonyl]), block copolymers as polyethyleneoxide/polypropyleneoxide block copolymers such as poloxamers, poloxamer 188 and poloxamer 407, Brij® 35, Brij® 56, Brij® 72, Brij® 76, Brij® 92V, Brij® 97, Brij® 58P, Cremophor® EL, Decaethylene glycol monododecyl ether, N-Decanoyl-N-methylglucamine, n-Dodecanoyl-N-methylglucamide, alkyl-polyglucosides, ethoxylated castor oil, Heptaethylene glycol monodecyl ether, Heptaethylene glycol monododecyl ether, Heptaethylene glycol monotetradecyl ether, Hexaethylene glycol monododecyl ether, Hexaethylene glycol monohexadecyl ether, Hexaethylene glycol monooctadecyl ether, Hexaethylene glycol monotetradecyl ether, Igepal CA-630, Igepal CA-630, Methyl-6-O-(N-heptylcarbamoyl)-beta-D-glucopyranoside, Nonaethylene glycol monododecyl ether, N-Nonanoyl-N-methylglucamine, N-Nonanoyl-N-methylglucamine, Octaethylene glycol monodecyl ether, Octaethylene glycol monododecyl ether, Octaethylene glycol monohexadecyl ether, Octaethylene glycol monooctadecyl ether, Octaethylene glycol monotetradecyl ether, Octyl-β-D-glucopyranoside, Pentaethylene glycol monodecyl ether, Pentaethylene glycol monododecyl ether, Pentaethylene glycol monohexadecyl ether, Pentaethylene glycol monohexyl ether, Pentaethylene glycol monooctadecyl ether, Pentaethylene glycol monooctyl ether, Polyethylene glycol diglycidyl ether, Polyethylene glycol ether W-1, Polyoxyethylene 10 tridecyl ether, Polyoxyethylene 100 stearate, Polyoxyethylene 20 isohexadecyl ether, Polyoxyethylene 20 oleyl ether, Polyoxyethylene 40 stearate, Polyoxyethylene 50 stearate, Polyoxyethylene 8 stearate, Polyoxyethylene bis(imidazolyl carbonyl), Polyoxyethylene 25 propylene glycol stearate, Saponin from Quillaja bark, Span® 20, Span® 40, Span® 60, Span® 65, Span® 80, Span® 85, Tergitol, Type 15-S-12, Tergitol, Type 15-S-30, Tergitol, Type 15-S-5, Tergitol, Type 15-S-7, Tergitol, Type 15-S-9, Tergitol, Type NP-10, Tergitol, Type NP-4, Tergitol, Type NP-40, Tergitol, Type NP-7, Tergitol, Type NP-9, Tetradecyl-β-D-maltoside, Tetraethylene glycol monodecyl ether, Tetraethylene glycol monododecyl ether, Tetraethylene glycol monotetradecyl ether, Triethylene glycol monodecyl ether, Triethylene glycol monododecyl ether, Triethylene glycol monohexadecyl ether, Triethylene glycol monooctyl ether, Triethylene glycol monotetradecyl ether, Triton CF-21, Triton CF-32, Triton DF-12, Triton DF-16, Triton GR-5M, Triton QS-15, Triton QS-44, Triton X-100, Triton X-102, Triton X-15, Triton X-151, Triton X-200, Triton X-207, Triton® X-100, Triton® X-114, Triton® X-165 solution, Triton® X-305 solution, Triton® X-405, Triton® X-45, Triton® X-705-70, TWEEN® 20, TWEEN® 40, TWEEN® 60, TWEEN® 6, TWEEN® 65, TWEEN® 80, TWEEN® 81, TWEEN® 85, Tyloxapol, sphingophospholipids (sphingomyelin), Solutol HS 15 (also known as Macrogol 15 Hydroxystearate) and d-alfa-Tocopheryl polyethylene glycol 1000 succinate (also known as vitamin E TPGS or TPGS), and sphingoglycolipids (ceramides, gangliosides), phospholipids, and/or n-Undecyl β-D-glucopyranoside.

In one aspect of the invention, the surfactant is a zwitterionic surfactant.

Zwitterionic surfactants may be selected from the group of: CHAPS, CHAPSO, 3-(Decyldimethylammonio) propanesulfonate inner salt, 3-(Dodecyldimethylammonio)-propanesulfonate inner salt, 3-(Dodecyldimethylammonio) propanesulfonate inner salt, 3-(N,N-Dimethylmyristylammonio)propanesulfonate, 3-(N,N-Dimethyloctadecylammonio)-propanesulfonate, 3-(N,N-Dimethyloctylammonio)propanesulfonate inner salt, 3-(N,N-Dimethylpalmitylammonio) propanesulfonate, N-alkyl-N,N-dimethylammonio-1-propanesulfonates, 3-cholamido-1-propyldimethylammonio-l-propanesulfonate, Dodecylphosphocholine, myristoyl lysophosphatidylcholine, Zwittergent 3-12 (N-dodecyl-N,N-dimethyl-3-ammonio-1-propanesulfonate), Zwittergent 3-10 (3-(Decyldimethylammonio)-propanesulfonate inner salt), Zwittergent 3-08 (3-(Octyldimethylammonio)propanesulfonate), glycerophospholipids (lecithins, kephalins, phosphatidyl serine), glyceroglycolipids (galactopyranoside), alkyl, alkoxyl (alkyl ester), alkoxy (alkyl ether)-derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, lysophosphatidylserine and lysophosphatidylthreonine, acylcarnitines and derivatives, $N^{beta}$-acylated derivatives of lysine, arginine or histidine, or side-chain acylated derivatives of lysine or arginine, $N^{beta}$-acylated derivatives of dipeptides comprising any combination of lysine, arginine or histidine and a neutral or acidic amino acid, $N^{beta}$-acylated derivative of a tripeptide comprising any combination of a neutral amino acid and two charged amino acids, or the surfactant may be selected from the group of imidazoline derivatives, long-chain fatty acids and salts thereof $C_6$-$C_{12}$ (eg. oleic acid and caprylic acid), N-Hexadecyl-N,N-dimethyl-3-ammonio-1-propanesulfonate, anionic (alkyl-aryl-sulphonates) monovalent surfactants, palmitoyl lysophosphatidyl-L-serine, lysophospholipids (e.g. 1-acyl-sn-glycero-3-phosphate esters of ethanolamine, choline, serine or threonine), or mixtures thereof.

In one aspect of the invention, the surfactant is an alkyl-polyglucoside. The term "alkyl-polyglucosides" as used herein in relates to a straight or branched $C_{5-20}$-alkyl, -alkenyl or - alkynyl chain which is substituted by one or more glucoside moieties such as maltoside, saccharide etc. Embodiments of these alkyl-polyglucosides include $C_{6-18}$-alkyl-polyglucosides. Specific embodiments of these alkyl-polyglucosides includes the even numbered carbonchains such as $C_6$, $C_8$, $C_{10}$, $C_{12}$, $C_{14}$, $C_{16}$, $C_{18}$ and $C_{20}$ alkyl chain. Specific embodiments of the glucoside moieties include pyranoside, glucopyranoside, maltoside, maltotrioside and sucrose. In embodiments of the invention, less than 6 glucosid moieties are attached to the alkyl group. In embodiments of the invention, less than 5 glucosid moieties are attached to the alkyl group. In embodiments of the invention, less than 4 glucosid moieties are attached to the alkyl group. In embodiments of the invention, less than 3 glucosid moieties are attached to the alkyl group. In embodiments of the invention, less than 2 glucosid moieties are attached to the alkyl group. Specific embodiments of alkyl-polyglucosides are alkyl glucosides such n-decyl β-D-glucopyranoside, decyl β-D-maltopyranoside, dodecyl β-D-glucopyranoside, n-dodecyl β-D-maltoside, tetradecyl β-D-glucopyranoside, decyl β-D-maltoside, hexadecyl β-D-maltoside, decyl β-D-maltotrioside, dodecyl β-D-maltotrioside, tetradecyl β-D-maltotrioside, hexadecyl β-D-maltotrioside, n-dodecyl-sucrose, n-decylsucrose, sucrose monocaprate, sucrose monolaurate, sucrose monomyristate, and sucrose monopalmitate.

In one aspect of the invention, the surfactant is Tetradecyl-β-D-maltoside or n-dodecyl β-D-maltoside.

In one aspect of the invention, said surfactant is a poloxamer. In a further aspect of the invention, said surfactant is a poloxamer 188. In a further aspect of the invention, said surfactant is selected from the group consisting of poloxamer 407, poloxamer 124, poloxamer 181, poloxamer 182, poloxamer 237, poloxamer 331 and poloxamer 338. In a further aspect of the invention, said surfactant is a polysorbate 20 (Tween-20). In a further aspect of the invention, said surfactant is a polysorbate 80. In a further aspect of the invention, said surfactant is a Solutol HS 15. In a further aspect of the invention, said surfactant is a d-alfa-Tocopheryl polyethylene glycol 1000 succinate. In a further aspect of the invention, said surfactant a Tetradecyl-β-D-maltoside.

In one aspect of the invention, the concentration of said surfactant is from about 5 mg/L to about 50 g/L. In a further aspect of the invention, the concentration of said surfactant is from about 10 mg/L to about 30 g/L. In a further aspect of the invention, the concentration of said surfactant is from about 20 mg/L to about 3000 mg/L. In a further aspect of the invention, the concentration of said surfactant is from about 30 mg/L to about 500 mg/L. In a further aspect of the invention, the concentration of said surfactant is from about 50 mg/L to about 200 mg/L.

In one aspect of the invention, the surfactant is added in a concentration between 0.1 mM and 10 mM, preferably between 0.5 mM and 5 mM, preferably between 0.5 mM and 3 mM.

In one aspect of the invention, the pharmaceutical composition comprises two different surfactants. In one aspect of the invention, said surfactants are poloxamer 188 and polysorbate 20 (Tween-20). In one aspect of the invention, at least one of the two different surfactants is an anionic surfactant.

In one aspect of the invention, the pharmaceutical composition further comprises a protamine salt, wherein said protamine salt is present in said composition in a concentration of greater than 0.25mM and wherein said composition has a pH of less than about 7.0. In the compositions of the invention, wherein a protamine salt is to be included the pH of said pharmaceutical composition or a reconstituted solution of said pharmaceutical composition is in one aspect from about pH 3.0 to about pH 6.0, in a further aspect from about pH 4.0 to about pH 5.5, in a further aspect from about pH 4.0 to about pH 5.0, in a further aspect from about pH 3.0 to about pH 4.0, and in yet a further aspect from about pH 4.0 to about pH 5.5.

In the compositions of the invention, wherein a protamine salt is to be included it is to be a protamine salt other than protamine sulphate where such salts include, but are not limited to, acetate, bromide , chloride, caproate, trifluoroacetate, $HCO_3$, propionate, lactate, formiate, nitrate, citrate, monohydrogenphosphate, dihydrogenphosphate, tartrate, or perchlorate salts of protamine or mixtures of any two protamine salts. "Protamine" as used herein refers to the generic name of a group of strongly basic proteins present in sperm cells in salt-like combination with nucleic acids. Normally, protamines to be used are obtained from e.g. salmon (salmine), rainbow trout (iridine), herring (clupeine), sturgeon (sturine), orspanish mackerel or tuna (thynnine) and a wide variety of salts of protamines are commercially available. Of course, it is understood that the peptide composition of a specific protamine may vary depending of which family, genera or species of fish it is obtained from. Protamine usually contains four major components, i.e. single-chain peptides containing about 30-32 residues of which about 21-22 are arginines. The N-terminal is proline for each of the four main components, and since no other amino groups are present in the sequence, chemical modification of protamine by a particular salt is expected to be homogenous in this context.

In one embodiment, the protamine salts used in the present invention are from salmon.

In another embodiment, the protamine salts used in the present invention are from herring.

In another embodiment, the protamine salts used in the present invention are from rainbow trout.

In another embodiment, the protamine salts used in the present invention are from tuna.

In another embodiment, the protamine salt is selected from the group consisting of propionate, lactate, formiate, nitrate, acetate, citrate, caproate, monohydrogenphosphate, dihydrogenphosphate salts of protamine.

In another embodiment, the protamine salt is selected from the group consisting of propionate, lactate, formiate, nitrate and acetate salts of protamine.

In another embodiment, the protamine salt is selected from acetate salts of protamine.

In the compositions of the invention, wherein a protamine salt is to be included, a surfactant may further be included. In one aspect of the invention, such a surfactant is poloxamer 188. In a further aspect of the invention, such a surfactant is selected from the group consisting of poloxamer 407, poloxamer 124, poloxamer 181, poloxamer 182, poloxamer 237, poloxamer 331 and poloxamer 338. In a further aspect, such a surfactant is polysorbate 20 (Tween-20). In a further aspect such a surfactant is a polysorbate 80. In a further aspect, such a surfactant is a Solutol HS 15. In a further aspect, such a surfactant is a d-alfa-Tocopheryl polyethylene glycol 1000 succinate. In a further aspect, such a surfactant is a Tetradecyl-$\beta$-D-maltoside.

In a further embodiment, when protamine salt is to be included in the composition of the invention it is to be a mixture of two different salts, one salt will be acetate and the other salt is selected from the group consisting of propionate, lactate, formiate, and nitrate salts of protamine. It is to be understood that when the protamine salt to be included in the formulation of the invention is to be a mixture of two different salts, the molar ratio between the two different salts may be from 0.1:1 to 1:1.

In a further embodiment, when protamine salt is to be included in the composition of the invention the meal-related insulin is selected from the group consisting of human insulin, human insulin$^{B28D}$, human insulin$^{B28K, B29P}$, and insulin glulisine. In yet a further embodiment, the meal-related insulin peptide is human insulin. In yet a further embodiment, the meal-related insulin peptide is human insulin$^{B28D}$. In yet a further embodiment, the meal-related insulin peptide is human insulin$^{B28K, B29P}$.

In one embodiment, the molar ratio of protamine salt to insulin peptide in the compositions of the invention is from about 0.5 to about 100.

In another embodiment, the molar ratio of of protamine salt to insulin peptide in the compositions of the invention is from about 0.5 to about 10.

In another embodiment, the molar ratio of protamine salt to insulin peptide in the compositions of the invention is from about 0.5 to 5.

In another embodiment, the molar ratio of protamine salt to insulin peptide in the compositions of the invention is from about 1 to 3.

In another aspect, the invention relates to a method for treatment of hyperglycemia by parenteral administration of an effective amount of a pharmaceutical composition, which comprises an amylin peptide, and a meal-related insulin peptide.

In another aspect, the present invention relates to a method for treatment of binge eating or bulimia comprising parenteral administration of an effective amount of a pharmaceutical composition, which comprises an amylin peptide, and a meal-related insulin peptide.

In another aspect, the present invention relates to a method for treatment or prevention of type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, stroke, inflammatory bowel syndrome, dyspepsia and gastric ulcers comprising parenteral administration of an effective amount of a pharmaceutical composition, which comprises an amylin peptide, and a meal-related insulin peptide.

In another aspect, the present invention relates to a method for delaying or preventing disease progression in type 2 diabetes comprising parenteral administration of an effective amount of a pharmaceutical composition, which comprises an amylin peptide, and a meal-related insulin peptide.

In another aspect, the present invention relates to a method for decreasing food intake, decreasing $\beta$-cell apoptosis, increasing $\beta$-cell function and $\beta$-cell mass, and/or for restoring glucose sensitivity to $\beta$-cells comprising parenteral administration of an effective amount of a pharmaceutical composition, which comprises an amylin peptide, and a meal-related insulin peptide.

In a further aspect the present invention relates to a method of treating any of the above conditions which further comprises administering to a person in need thereof a pharmaceutically relevant amount of GLP-1 or a GLP-1 derivative. In another embodiment the GLP-1 derivative to be employed in combination with a composition of the present invention refers to GLP-1(1-37), exendin-4(1-39), insulinotropic fragments thereof, insulinotropic analogues thereof and insulinotropic derivatives thereof. Insulinotropic fragments of GLP-1(1-37) are insulinotropic peptides for which the entire sequence can be found in the sequence of GLP-1(1-37) and where at least one terminal amino acid has been deleted. Examples of insulinotropic fragments of GLP-1(1-37) are GLP-1(7-37) wherein the amino acid residues in positions 1-6 of GLP-1(1-37) have been deleted, and GLP-1(7-36) where the amino acid residues in position 1-6 and 37 of GLP-1 (1-37) have been deleted. Examples of insulinotropic fragments of exendin-4(1-39) are exendin-4(1-38) and exendin-4 (1-31). The insulinotropic property of a compound may be determined by in vivo or in vitro assays well known in the art. For instance, the compound may be administered to an animal and monitoring the insulin concentration over time. Insulinotropic analogues of GLP-1(1-37) and exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound. Examples of insulinotropic analogues of GLP-1(1-37) are e.g. $Met^8$-GLP-1(7-37) wherein the alanine in position 8 has been replaced by methionine and the amino acid residues in position 1 to 6 have been deleted, and $Arg^{34}$-GLP-1(7-37) wherein the valine in position 34 has been replaced with arginine and the amino acid residues in position 1 to 6 have been deleted. An example of an insulinotropic analogue of exendin-4(1-39) is $Ser^2Asp^3$-exendin-4(1-39) wherein the amino acid residues in position 2 and 3 have been replaced with serine and aspartic acid, respectively (this particular analogue also being known in the art as exendin-3). Insulinotropic derivatives of GLP-1(1-37), exendin-4(1-39) and analogues thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups and lipophilic substituents. Examples of insulinotropic derivatives of GLP-1(1-37), exendin-4(1-39) and analogues thereof are GLP-1(7-36)-amide, $Arg^{34}$, $Lys^{26}$ $(N^\varepsilon$-$(\gamma$-Glu$(N^\alpha$-hexadecanoyl$)))$-GLP-1(7-37) and $Tyr^{31}$-exendin-4(1-31)-amide. Further examples of GLP-1(1-37), exendin-4(1-39), insulinotropic fragments thereof, insulinotropic analogues thereof and insulinotropic derivatives thereof are described in WO 98/08871, WO 99/43706, US 5424286 and WO 00/09666.

When the pharmaceutical compositions according to the present invention are administered by injection, e.g. via a pen or a syringe, it is typically administered 3 times per day, preferably before meals. It is preferred that each administration comprises less than about 500 µL, or less than about 200 µL since larger injection volumes are unpleasant for the patient. When the pharmaceutical compositions according to the present invention are administered by a pump, it is typically administrated continuously or discontinuously such as via at least 10 administrations or more per day.

In one embodiment of the invention, the method of treatment comprises administration of an effective amount of the pharmaceutical composition which is from 30 µL/day to about 600 µL/day, such as from about 60 µL/day to about 360 µL/day. In another embodiment of the invention the method comprises a pharmaceutical composition for administration by subcutaneous injection. In another embodiment of the invention, the method comprises a pharmaceutical composition for administration by a pump. In another embodiment of the invention, the method comprises administration by a pump which delivers a discontinuous amount of said pharmaceutical composition. In another embodiment of the invention, the method comprises administration by a pump which delivers a discontinuous amount of said pharmaceutical composition wherein said discontinuous administration of said pharmaceutical composition is by a pulse dosing for a period of time which is less than the period between pulses.

In another aspect the present invention, relates to the use of an amylin peptide and a meal-related insulin peptide for the manufacture of a pharmaceutical composition for parenteral administration, which comprises an amylin peptide, and a meal-related insulin peptide. In one embodiment of the invention, the use comprises a pharmaceutical composition for administration by subcutaneous injection. In another embodiment of the invention, the use comprises a pharmaceutical composition for administration by a pump. In another embodiment of the invention, the use comprises administration by a pump which delivers a discontinuous amount of said pharmaceutical composition. In another embodiment of the invention, the use comprises administration by a pump which delivers a discontinuous amount of said pharmaceutical composition wherein said discontinuous administration of said pharmaceutical composition is by a pulse dosing for a period of time which is less than the period between pulses.

In another aspect, the present invention relates to the use of an amylin peptide and a meal-related insulin peptide for the manufacture of a pharmaceutical composition for the treatment of hyperglycemia by parenteral administration, which composition comprises an an amylin peptide and a meal-related insulin peptide.

In another aspect, the present invention relates to pharmaceutical composition according to the invention for use in treatment of hyperglycemia. In another aspect, the present invention relates to the use of an amylin peptide and a meal-related insulin peptide for the manufacture of a pharmaceutical composition for the treatment of binge eating or bulimia.

FURTHER EMBODIMENTS OF THE INVENTION

1. A soluble pharmaceutical composition for parenteral administration, which comprises an amylin peptide, and a meal-related insulin peptide.

2. The pharmaceutical composition as defined in embodiment 1, wherein the pH of said pharmaceutical composition or a reconstituted solution of said pharmaceutical composition is from about pH 6.5 to about pH 9.0.

3. The pharmaceutical composition as defined in embodiment 1, wherein the pH of said pharmaceutical composition or a reconstituted solution of said pharmaceutical composition is from about pH 6.8 to about pH 8.0.

4. The pharmaceutical composition as defined in embodiment 1, wherein the pH of said pharmaceutical composition or a reconstituted solution of said pharmaceutical composition is from about pH 7.0 to about pH 7.8.

5. The pharmaceutical composition as defined in embodiment 1, wherein the pH of said pharmaceutical composition or a reconstituted solution of said pharmaceutical composition is from about pH 7.2 to about pH 7.6.

6. The pharmaceutical composition as defined in any of the embodiments 1-5, wherein the composition is a solution.

7. The pharmaceutical composition as defined in any of the embodiments 1-5, wherein the composition is a solid.

8. The pharmaceutical composition as defined in embodiment 7, which is to be reconstituted with an aqueous solution, such as a buffer or water for injection.

9. The pharmaceutical composition as defined in any of the embodiments 1-8, which is suitable for administration by injection or infusion.

10. The pharmaceutical composition as defined in any of the embodiments 1-9, wherein said meal-related insulin peptide has a time action of less than 4 hours.

11. The pharmaceutical composition as defined in any of the embodiments 1-10, wherein said meal-related insulin peptide is human insulin, an analog of human insulin, a derivative of human insulin or a derivative of a human insulin analog.

12. The pharmaceutical composition as defined in embodiment 11, wherein said meal-related insulin peptide is human insulin.

13. The pharmaceutical composition as defined in embodiment 11, wherein said meal-related insulin peptide is a human insulin analog.

14. The pharmaceutical composition as defined in embodiment 13, wherein said meal-related human insulin analog is human insulin$^{B28D}$.

15. The pharmaceutical composition as defined in embodiment 13, wherein said meal-related human insulin analog is human insulin$^{B28K, B29P}$.

16. The pharmaceutical composition as defined in embodiment 13, wherein said meal-related human insulin analog is human insulin$^{B3K, B29E}$.

17. The pharmaceutical composition as defined in embodiment 13, wherein said meal-related human insulin analog is human insulin $^{desB30}$.

18. The pharmaceutical composition as defined in embodiment 13, wherein said meal-related insulin peptide is a derivative of a human insulin analog.

19. The pharmaceutical composition as defined in any of the embodiments 1-18, wherein the concentration of said meal-related insulin peptide is in the range from about 1.6 mg/mL to about 5.6 mg/mL, or from about 2.6 mg/mL to about 4.6 mg/mL, or from about 3.2 mg/mL to about 4.0 mg/mL.

20. The pharmaceutical composition as defined in any of the embodiments 1-18, wherein the concentration of said meal-related insulin peptide is in the range from about 1 mg/mL to about 10 mg/mL, or from about 2.5 mg/mL to about 8.75 mg/mL, or from about 3.5 mg/mL to about 8.75 mg/mL, or from about 5 mg/mL to about 8.75 mg/mL.

21. The pharmaceutical composition as defined in any of the embodiments 1-20, comprising two different insulin peptides.

22. The pharmaceutical composition as defined in any of the embodiments 1-21, wherein said amylin peptide is amylin, an amylin analog or an amylin agonist.

23. The pharmaceutical composition as defined in any of the embodiments 1-22, wherein said amylin peptide is human amylin.

24. The pharmaceutical composition as defined in any of the embodiments 1-23, wherein said amylin peptide is $^{25,28,29}$Pro- h-amylin.

25. The pharmaceutical composition as defined in any of the embodiments 1-24, wherein said amylin peptide is human amylin methylated in position 24 and 26.

26. The pharmaceutical composition as defined in any of the embodiments 1-24, wherein said meal-related insulin peptide is human insulin and said amylin peptide is $^{25,28,29}$Pro- h-amylin.

27. The pharmaceutical composition as defined in any of the embodiments 1-26, wherein the concentration of said amylin peptide is in the range from about 0.05 mg/mL to about 10 mg/mL or from about 0.1 mg/mL to about 4 mg/mL, or from about 0.4 mg/mL to about 1.2 mg/mL.

28. The pharmaceutical composition as defined in any of the embodiments 1-27, wherein the concentration of said amylin peptide is in the range from about 0.05 mg/mL to about 10 mg/mL and the concentration of human insulin is in the range from about 3.2 mg/mL to about 4.0 mg/mL.

29. The pharmaceutical composition as defined in any of the embodiments 1-28, wherein said insulin peptide is human insulin$^{B28D}$ and said amylin peptide is $^{25,28,29}$Pro- h-amylin.

30. The pharmaceutical composition as defined in embodiment 29, wherein the concentration of $^{25,28,29}$Pro- h-amylin is in the range from about 0.05 mg/mL to about 10 mg/mL and the concentration of human insulin$^{B28D}$ is in the range from about 0.3 mg/mL to about 4.0 mg/mL.

31. The pharmaceutical composition as defined in embodiment 30, wherein the concentration of $^{25,28,29}$Pro- h-amylin is in the range from about 0.1 mg/mL to about 4 mg/mL,and the concentration of human insulin$^{B28D}$ is in the range from about 0.36 mg/mL to about 3.8 mg/mL.

32. The pharmaceutical composition as defined in any of the embodiments 1-31, wherein said insulin peptide is human insulin$^{B3K, B29E}$ and said amylin peptide is $^{25,28,29}$Pro- h-amylin.

33. The pharmaceutical composition as defined in embodiment 32, wherein the concentration of human insulin$^{B3K, B29E}$ is in the range from about 0.36 mg/mL to about 4.0 mg/mL.

34. The pharmaceutical composition as defined in any of the embodiments 1-33, comprising zinc and/or calcium.

35. The pharmaceutical composition as defined in embodiment 34, wherein the molar ratio of zinc to insulin peptide is from 1/6 to ½ mole/mole, preferable from 3/12 to 5/12 mole/mole.

36. The pharmaceutical composition as defined in embodiment 34, wherein the molar ratio of calcium to insulin peptide is from 3/12 to 5/12 mole/mole, preferable from 1/6 to 1/3 mole/mole.

37. The pharmaceutical composition as defined in any of the embodiments 1-36, wherein said pharmaceutical composition comprises a preservative.

38. The pharmaceutical composition as defined in any of the embodiments 1-37, wherein said preservative is selected from the group consisting of phenol, m-cresol or a mixture thereof.

39. The pharmaceutical composition as defined in any of the embodiments 1-38, wherein said pharmaceutical composition comprises a buffer.

40. The pharmaceutical composition as defined in embodiment 39, wherein said buffer is selected from the group consisting of phosphate, a Good's buffer such as TRIS, BICINE, and HEPES, glycine, glycylglycine, citrate or a mixture thereof.

41. The pharmaceutical composition as defined in any of the embodiments 1-40, wherein said pharmaceutical composition comprises an isotonicity agent.

42. The pharmaceutical composition as defined in embodiment 41, wherein said isotonicity agent is not a salt.

43. The pharmaceutical composition as defined in embodiment 41, wherein said isotonicity agent is selected from the group consisting of trehalose, glucose, mannitol, sorbitol, glycerol, propylene glycol or a mixture thereof.

44. The pharmaceutical composition as defined in any of the embodiments 1-43, which further comprises a stabiliser.

45. The pharmaceutical composition as defined in embodiment 44, wherein said stabiliser is selected from the group consisting of L-histidine, imidazole and L-arginine.

46. The pharmaceutical composition as defined in embodiment 44, wherein said stabiliser is a polyethylene glycol.

47. The pharmaceutical composition as defined in any of the embodiments 1-46, which further comprises a surfactant.

48. The pharmaceutical composition as defined in embodiment 47, wherein said surfactant is a poloxamer.

49. The pharmaceutical composition as defined in embodiment 48, wherein said surfactant is a poloxamer 188.

50. The pharmaceutical composition as defined in embodiment 48, wherein said surfactant is selected from the group consisting of poloxamer 407, poloxamer 124, poloxamer 181, poloxamer 182, poloxamer 237, poloxamer 331 and poloxamer 338.

51. The pharmaceutical composition as defined in embodiment 47, wherein said surfactant is a polysorbate 20 (Tween-20).

52. The pharmaceutical composition as defined in embodiment 47, wherein said surfactant is a polysorbate 80.

53. The pharmaceutical composition as defined in embodiment 47, wherein said surfactant is a Solutol HS 15.

54. The pharmaceutical composition as defined in embodiment 47, wherein said surfactant is a d-alfa-Tocopheryl polyethylene glycol 1000 succinate.

55. The pharmaceutical composition as defined in embodiment 47, wherein said surfactant a Tetradecyl-β-D-maltoside or n-dodecyl β-D-maltoside.

56. The pharmaceutical composition as defined in any of the embodiments 47-55, wherein the concentration of said surfactant is from about 5 mg/L to about 50 g/L.

57. The pharmaceutical composition as defined in any of the embodiments 47-56, wherein the concentration of said surfactant is from about 10 mg/L to about 30 g/L.

58. The pharmaceutical composition as defined in any of the embodiments 47-57, wherein the concentration of said surfactant is from about 20 mg/L to about 3000 mg/L.

59. The pharmaceutical composition as defined in any of the embodiments 47-58, wherein the concentration of said surfactant is from about 30 mg/L to about 500 mg/L.

60. The pharmaceutical composition as defined in any of the embodiments 47-59, wherein the concentration of said surfactant is from about 50 mg/L to about 200 mg/L.

61. The pharmaceutical composition as defined in any of the embodiments 47-60, comprising two different surfactants.

62. The pharmaceutical composition as defined in embodiment 61, comprising poloxamer 188 and polysorbate 20 (Tween-20).

63. The pharmaceutical composition as defined in embodiment 1, further comprising a protamine salt, wherein said protamine salt is present in said formulation in a concentration of greater than 0.25mM and wherein said formulation has a pH of less than about 7.0.

64. The pharmaceutical composition as defined in embodiment 63, wherein the pH of said pharmaceutical composition or a reconstituted solution of said pharmaceutical composition is from about pH 3.0 to about pH 6.0.

65. The pharmaceutical composition as defined in embodiment 63, wherein the pH of said pharmaceutical composition or a reconstituted solution of said pharmaceutical composition is from about pH 4.0 to about pH 5.5.

66. The pharmaceutical composition as defined in embodiment 63, wherein the pH of said pharmaceutical composition or a reconstituted solution of said pharmaceutical composition is from about pH 4.0 to about pH 5.0.

67. The pharmaceutical composition as defined in embodiment 63, wherein the pH of said pharmaceutical composition or a reconstituted solution of said pharmaceutical composition is from about pH 3.0 to about pH 4.0.

68. The pharmaceutical composition as defined in embodiment 63, wherein the pH of said pharmaceutical composition or a reconstituted solution of said pharmaceutical composition is from about pH 4.0 to about pH 5.5.

69. The pharmaceutical composition as defined in any of the embodiments 63-68, wherein the molar ratio of protamine salt to insulin is from about 0.5 to about 100.

70. The pharmaceutical composition as defined in embodiment 69, wherein the molar ratio of protamine salt to insulin is from about 0.5 to about 10.

71. The pharmaceutical composition as defined in embodiment 70, wherein the molar ratio of protamine salt to insulin is from about 0.5 to 5.

72. The pharmaceutical composition as defined in any of the embodiments 63-71, wherein the protamine salt is selected from the group consisting of propionate, lactate, formiate, nitrate and acetate salts of protamine.

73. The pharmaceutical composition as defined in embodiment 72, wherein the protamine salt is protamine acetate.

74. The pharmaceutical composition as defined in any of the embodiments 63-73, as further defined in any of the embodiments 1-62.

75. The pharmaceutical composition as defined in embodiment 74, wherein said surfactant is a poloxamer 188.

76. The pharmaceutical composition as defined in embodiment 74, wherein said surfactant is selected from the group consisting of poloxamer 407, poloxamer 124, poloxamer 181, poloxamer 182, poloxamer 237, poloxamer 331 and poloxamer 338.

77. The pharmaceutical composition as defined in embodiment 74, wherein said surfactant is a polysorbate 20 (Tween-20).

78. The pharmaceutical composition as defined in embodiment 74, wherein said surfactant is a polysorbate 80.

79. The pharmaceutical composition as defined in embodiment 74, wherein said surfactant is a Solutol HS 15.

80. The pharmaceutical composition as defined in embodiment 74, wherein said surfactant is a d-alfa-Tocopheryl polyethylene glycol 1000 succinate.

81. The pharmaceutical composition as defined in embodiment 74, wherein said surfactant a Tetradecyl-β-D-maltoside.

82. The pharmaceutical composition as defined in any of the embodiments 63-81, wherein the meal-related insulin is selected from the group consisting of human insulin, human insulin$^{B28D}$, human insulin$^{B28K, B29P}$ and insulin glulisine.

83. The pharmaceutical composition as defined in embodiment 82, wherein said meal-related insulin peptide is human insulin.

84. The pharmaceutical composition as defined in embodiment 82, wherein said meal-related human insulin analog is human insulin$^{B28D}$.

85. The pharmaceutical composition as defined in embodiment 82, wherein said meal-related human insulin analog is human insulin$^{B28K, B29P}$.

86. A method for treatment of hyperglycemia comprising parenteral administration of an effective amount of the pharmaceutical composition as defined in any of the embodiments 1-85.

87. The method as defined in embodiment 86, wherein said effective amount of the pharmaceutical composition is from about 30 μL/day to about 600 μL/day, such as from about 60 μL/day to about 360 μL/day.

88. The method as defined in any of the embodiments 86-87, wherein administration is by subcutaneous injection.

89. The method as defined in any of the embodiments 86-88, wherein administration is by a pump.

90. The method as defined in any of the embodiments 86-89, wherein administration is by a pump which delivers a discontinuous amount of said pharmaceutical composition.

91. The method as defined in embodiment 90, wherein said discontinuous administration of said pharmaceutical composition is by a pulse dosing for a period of time which is less than the period of time between pulses.

92. Use of a meal-related insulin peptide and an amylin peptide for the manufacture of a pharmaceutical composition as defined in any of the embodiments 1-85.

93. The use as defined in embodiment 92, wherein said pharmaceutical composition is for subcutaneous injection.

94. The use according as defined in any of the embodiments 92-93, wherein administration is by a pump.

95. The use as defined in any of the embodiments 92-94, wherein administration is by a pump which delivers a discontinuous amount of said pharmaceutical composition.

96. The use as defined in embodiment 95, wherein said discontinuous administration of said pharmaceutical composition is by a pulse dosing for a period of time which is less than the period of time between pulses.

97. Use of an insulin peptide and an amylin peptide for the manufacture of a pharmaceutical composition as defined in any of the embodiments 1-85 for the treatment of hyperglycemia.

98. Use of an insulin peptide and an amylin peptide for the manufacture of a pharmaceutical composition as defined in any of the embodiments 1-85 for the treatment of binge eating or bulimia.

The invention is also summarized in the following paragraphs:

1. A soluble pharmaceutical composition for parenteral administration, which comprises an amylin peptide, and a meal-related insulin peptide.

2. The pharmaceutical composition according to paragraph 1, wherein the pH of said pharmaceutical composition or a reconstituted solution of said pharmaceutical composition is from about pH 6.5 to about pH 9.0.

3. The pharmaceutical composition according to any one of the preceding paragraphs, wherein said meal-related insulin peptide is human insulin, an analog of human insulin, a derivative of human insulin or a derivative of a human insulin analog.

4. The pharmaceutical composition according to paragraph 3, wherein said meal-related human insulin analog is human insulin$^{B28D}$.

5. The pharmaceutical composition according to paragraph 3, wherein said meal-related human insulin analog is human insulin$^{B3K, B29E}$.

6. The pharmaceutical composition according to paragraph 3, wherein said meal-related human insulin analog is human insulin$^{B28K, B29P}$.

7. The pharmaceutical composition according to any of the preceding paragraphs, wherein the concentration of said meal-related insulin peptide is in the range from about 1.2 mg/mL to about 5.6 mg/mL.

8. The pharmaceutical composition according to any of the preceding paragraphs, wherein said amylin peptide is amylin, an amylin analog or an amylin agonist.

9. The pharmaceutical composition according to any of the preceding paragraphs, wherein said amylin peptide is $^{25, 28, 29}$Pro- h-amylin.

10. The pharmaceutical composition according to any of the paragraphs 1-8, wherein said amylin peptide is human amylin methylated in position 24 and 26.

11. The pharmaceutical composition to any of paragraphs 1-9, wherein said insulin peptide is human insulin$^{B28D}$ and said amylin peptide is $^{25, 28, 29}$Pro- h-amylin.

12. The pharmaceutical composition to any of the paragraphs 1-9, wherein said insulin peptide is human insulin$^{B3K,B29E}$ and said amylin peptide is $^{25, 28, 29}$Pro- h-amylin.

13. The pharmaceutical composition to any of the paragraphs 1-9, wherein said insulin peptide is human insulin$^{B28K, B29P}$ and said amylin peptide is $^{25, 28, 29}$Pro- h-amylin.

14. The pharmaceutical composition according any of the paragraphs 1 and 3-13, wherein the pH of the said pharmaceutical composition or a reconstituted solution of said pharmaceutical composition is from about pH 2.5 to about pH 4.5.

15. The pharmaceutical composition according to any one of the paragraphs 1-3, 7-10 and 14, wherein said meal-related human insulin analog is insulin$^{A21G, B28D, des B30}$.

16. The pharmaceutical composition according to any one of the paragraphs 1-3, 7-10 and 14, wherein said meal-related human insulin analog is insulin$^{A21G, B28E, des B30}$.

17. The pharmaceutical composition according to any one of the preceding paragraphs, comprising zinc and/or calcium.

18. The pharmaceutical composition according to any one of the preceding paragraphs, wherein said pharmaceutical composition comprises a preservative.

19. The pharmaceutical composition according to any one of the preceding paragraphs, wherein said pharmaceutical composition comprises a buffer.

20. The pharmaceutical composition according to any one of the preceding paragraphs, wherein said pharmaceutical composition comprises an isotonicity agent.

21. The pharmaceutical composition according to any one of the preceding paragraphs, which further comprises a stabiliser.

22. The pharmaceutical composition according to any one of the preceding paragraphs, which further comprises a surfactant.

23. The pharmaceutical composition according to paragraph 22, wherein said surfactant is anionic.

24. The pharmaceutical composition according to paragraph 23, wherein said anionic surfactant is a glycerophosphoglycerol derivative.

25. The pharmaceutical composition according to paragraph 24, wherein said glycerophosphoglycerol derivative is a dimyristoyl derivative.

26. The pharmaceutical composition according to paragraph 24, wherein said said glycerophosphoglycerol derivative is 1,2-Dimyristoyl-sn-glycero-3-phospho-rac-(1-glycerol) (DMPG).

27. The pharmaceutical composition according to any of the paragraphs 22-26, wherein the surfactant is added in a concentration between 0.1 mM and 10 mM.

28. A pharmaceutical composition according to any of paragraphs 1-27 for use in treatment of hyperglycemia.

29. A method for treatment of hyperglycemia comprising parenteral administration of an effective amount of the pharmaceutical composition according to any one of the paragraphs 1-28.

The features disclosed in the foregoing description may, both separately and in any combination thereof, be material for realising the invention in diverse forms thereof.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way,

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

All methods described herein can be performed in any suitable order unless otherwise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents,

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a formulation described herein as comprising a particular element should be understood as also describing a formulation consisting of that element, unless otherwise stated or clearly contradicted by context).

This invention includes all modifications and equivalents of the subject matter recited in the aspects or claims presented herein to the maximum extent permitted by applicable law.

The present invention is further illustrated in the following representative methods and examples which are, however, not intended to limit the scope of the invention in any way.

**METHODS**

**ThT fibrillation assay**

Low physical stability of a peptide may lead to amyloid fibril formation, which is observed as well-ordered, thread-like macromolecular structures in the sample eventually resulting in gel formation. In a ThT fibrillation assay application a small molecule indicator probe is used.

Thioflavin T (ThT) has a distinct fluorescence signature when binding to fibrils (Naiki et al. (1989) Anal. Biochem. 177, 244-249; LeVine (1999) Methods. Enzymol. 309, 274-284).

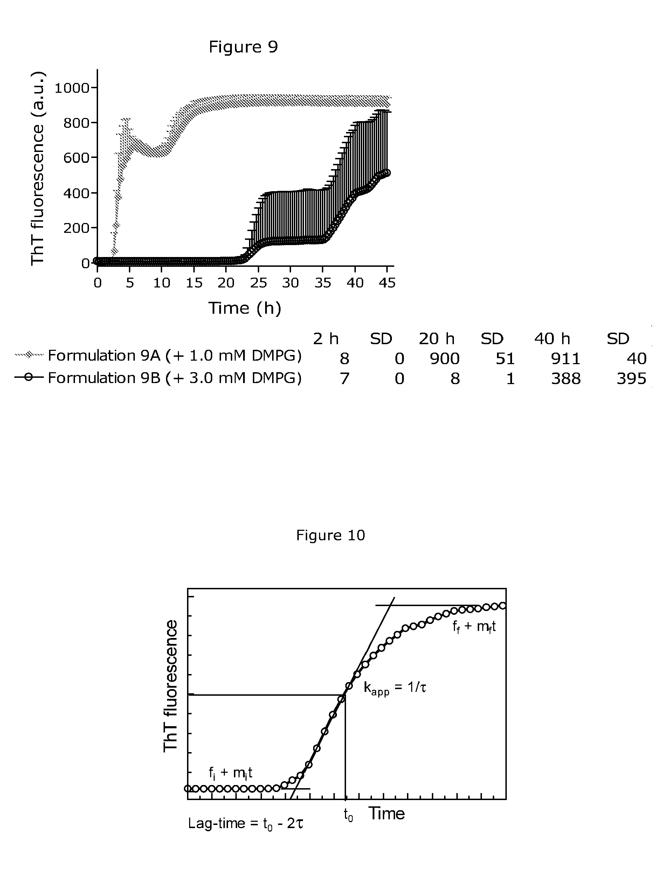

The time course for fibril formation can be described by a sigmoidal curve as shown in figure 10 with the following expression (Nielsen et al. (2001) Biochemistry 40, 6036-6046):

$$F = f_i + m_i t + \frac{f_f + m_f t}{1 + e^{-[(t-t_0)/\tau]}} \quad \text{Eq.(1)}$$

Here, F is the ThT fluorescence at the time t. The constant t0 is the time needed to reach 50% of maximum fluorescence. The two important parameters describing fibril formation are the lag-time calculated by t0 - 2τ and the apparent rate constant kapp = 1/τ.

Formation of a partially folded intermediate of the peptide is suggested as a general initiating mechanism for fibrillation. Few of those intermediates nucleate to form a template onto which further intermediates may assembly and the fibrillation proceeds. The lag-time corresponds to the interval in which the critical mass of nucleus is built up and the apparent rate constant is the rate with which the fibril itself is formed.

Sample preparation

Samples were prepared freshly before each assay. Each sample composition is described in the legends. The pH of the sample was adjusted to the desired value using appropriate amounts of concentrated NaOH and $HClO_4$. Thioflavin T was added to the samples from a stock solution in $H_2O$ to a final concentration of 1 μM.

Sample aliquots of 200 μl were placed in a 96 well microtiter plate (Packard OptiPlate™-96, white polystyrene). Usually, eight replica of each sample (corresponding to one test condition) were placed in one column of wells. The plate was sealed with Scotch Pad (Qiagen).

Incubation and fluorescence measurement

Incubation at given temperature, shaking and measurement of the ThT fluorescence emission were done in a Fluoroskan Ascent FL fluorescence platereader (Thermo Labsystems). The temperature was adjusted to 37 °C. The orbital shaking was adjusted to 960 rpm with an amplitude of 1 mm in all the presented data. Fluorescence measurement was done using excitation through a 444 nm filter and measurement of emission through a 485 nm filter. Each run was initiated by incubating the plate at the assay temperature for 10 min. The plate was measured every 20 minutes for a desired period of time. Between each measurement, the plate was shaken and heated as described.

Data handling

The measurement points were saved in Microsoft Excel format for further processing and curve drawing and fitting was performed using GraphPad Prism. The background emission from ThT in the absence of fibrils was negligible.

The data points are typically a mean of eight samples and shown with standard deviation error bars. Only data obtained in the same experiment (i.e. samples on the same plate) are presented in the same graph ensuring a relative measure of fibrillation between experiments.

The data set may be fitted to Eq. (1). However, since full sigmodial curves in this case are not always achieved during the measurement time, the degree of fibrillation is expressed as ThT fluorescence tabulated as the mean of the eight samples and shown with the standard deviation at various time points.

**Protein solubility**

The solubility of peptides and proteins depends on the pH of the solution. Often a protein or peptide precipitates at and/or close to its isoelectric point (pI), at which its netto charge is zero. At low pH (i.e. lower than the pI) proteins and peptides are typically positively charged, at pH higher than the pI they are typically negatively charged.

A prerequisite for coformulating a mixture consisting of both an insulin and an amylin molecule is that both peptides remain soluble in sufficient concentrations at a given pH, which is suitable for both formulating the drug product and for administrating the drug product to the patient e.g. by subcutaneous injection.

Solubility versus pH curves were measured in the following way. A formulation was prepared and aliquots were adjusted to pH values in the desired range by adding $HClO_4$ and NaOH. These samples were left equilibrating at room temperature for 2 - 3 days. Then the samples were centrifuged. A small aliquot of each sample was withdrawn for reverse HPLC analysis for determination of the concentration of the proteins in solution. The pH of each sample was measured after the centrifugation, and the concentration of each protein was depicted versus the measured pH in each sample.

**Amylin receptor binding assay**

For the receptor binding assay, membranes from the Amylin 3(a)/CRE-luc cells described below may be used. The tracer is Tyr-pramlintide iodinated with $^{125}I$ in the N-terminal tyrosine. SPA-WGA beads (GE Healthcare RPNQ0001) are incubated in a 96 well Optiplate in a buffer containing 50 mM Hepes, 5 mM $MgCl_2$, 5 mM EGTA, 0.025% Tween-20, pH 7.4 with membranes, tracer and a dilution series of the amylin analog.

After incubation for 2 hours at room temperature the plates are centrifuged and counted on a Topcounter. The EC50 is calculated as a measure of receptor affinity.

**Amylin luciferase assay**

1. Amylin assay outline

It has previously been published (Poyner DR et al 2002, Pharmacological Reviews 54(2) 233-246) that activation of Amylin receptors (coexpression of Calcitonin receptor and receptor activity modifying peptides RAMPs) by Amylin leads to an increase in the intracellular concentration of cAMP. Consequently, transcription is activated at promotors containing multiple copies of the cAMP response element (CRE). It is thus possible to measure Amylin activity by use of a CRE luciferase reporter gene introduced into BHK cells also expressing an Amylin receptor.

2. Construction of an Amylin 3(a)/CRE-luc cell line

A BHK570 cell line stably transfected with the human calcitonin receptor (CTa) and a CRE-responsive luciferase reportergene. The cell line was further transfected with RAMP-3, using standard methods. This turns the Calcitonin receptor into an Amylin 3(a) receptor. Methotrexate, Neomycin, and Hygromycin are selection markers for luciferase, the Calcitonin receptor, and RAMP-3, respectively.

3. Amylin luciferase assay

To perform activity assays, BHK Amylin 3(a)//CRE-luc cells are seeded in white 96 well culture plates at a density of about 20.000 cells/well. The cells are in 100 $\mu$l growth medium (DMEM with 10% FBS, 1% Pen/Strep, 1 mM Na-pyruvate, 250 nM Methotrexate, 500 $\mu$g/ml Neomycin, and 400 $\mu$g/ml Hygromycin). After incubation overnight at 37°C and 5% $CO_2$, the growth medium is replaced by 50 $\mu$l/well assay medium (DMEM (without phenol red), Glumamax™, 10% FBS, and 10 mM Hepes, pH 7,4). Further, 50 $\mu$l/well of standard or sample in assay buffer are added. After 4 hours incubation at 37°C and 5% $CO_2$, the assay medium with standard or sample are removed and replaced by 100 $\mu$l/well PBS. Further, 100$\mu$ l/well LucLite™ is added. The plates are sealed and incubated at room temperature for 30 minutes. Finally, luminescence is measured on a TopCounter (Packard) in SPC (single photon counting) mode.

**EXAMPLES**

In the following examples the above mentioned ThT fibrillation assay is used for assessment of physical stability of the formulations and the solubility is measured by the above mentioned solubility method.

**Example 1**

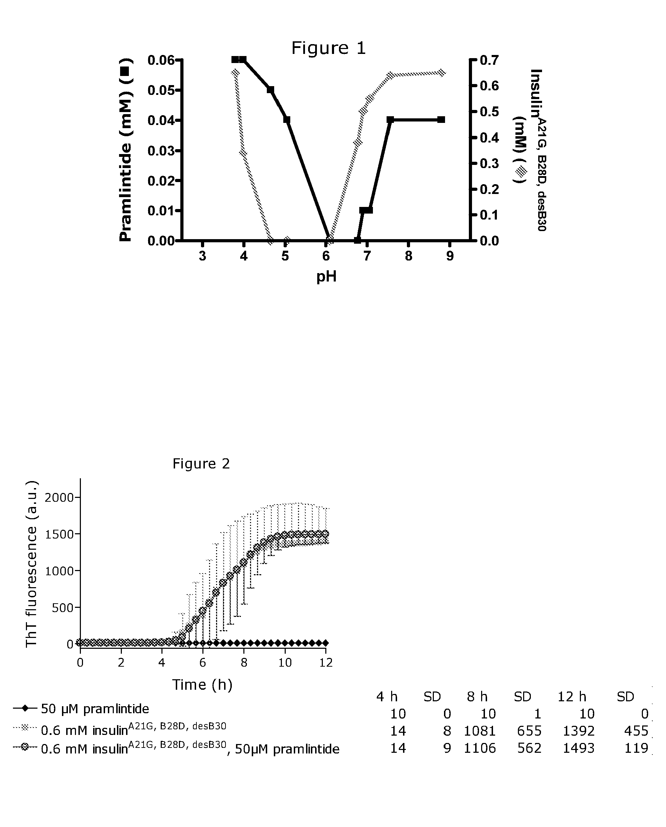

Figure 1 shows the solubility of a mixture of the amylin analogue pramlintide [25, 28, 29]Pro- h-amylin and insulin[A21G, B28D, desB30] versus pH. All samples contained 0.2 mM zinc-acetate, 16 mM m-cresol, 16 mM phenol. The concentration of [25,28,29]Pro- h-amylin in solution versus pH was plotted with a black line and symbols and using the left $\gamma$-axis; the concentration of the insulin analogue in solution versus pH was plotted with a light grey line and symbols and using the right y-axis. [25,28,29]Pro- h-amylin co-precipitated with the insulin analogue in its precipitation zone. At pH below 3.8 and above pH 7.5 substantial amounts of both peptides were soluble in this particular mix of analogues. This enables coformulation of therapeutically relevant doses of both insulin[A21G, B28D, desB30] and [25,28,29]Pro- h-amylin at acidic pH, e.g. pH 3.5.

**Example 2**

The physical stability of a mixture containing insulin[A21G, B28D, desB30] and pramlintide ([25, 28, 29]Pro-h-amylin) was assessed using a ThT fibrillation assay. This is shown in Figure 2. All three formulations contained 174 mM glycerol, 16 mM phenol, 16 mM m-cresol, 30 mM sodium acetate, and were adjusted to pH 3.5. Under these conditions [25, 28, 29]Pro-h-amylin was inert towards fibrillation throughout the assay time as this did not exhibit any ThT fluorescence signal. The insulin analogue alone fibrillated with a lag time of approx. 4.5 hours. The mixture with both the insulin analogue and [25,28,29]Pro- h-amylin exhibited the same ThT response (including the same lag time of approx. 4.5 hours) as the formulation with the insulin analogue alone. Hence, there was no mutual destabilisation since the mixture was just as physical stable as the least stable component (the insulin analogue) alone. This enables a stable coformulation of this insulin analogue and [25,28,29]Pro- h-amylin under these conditions.

**Example 3**

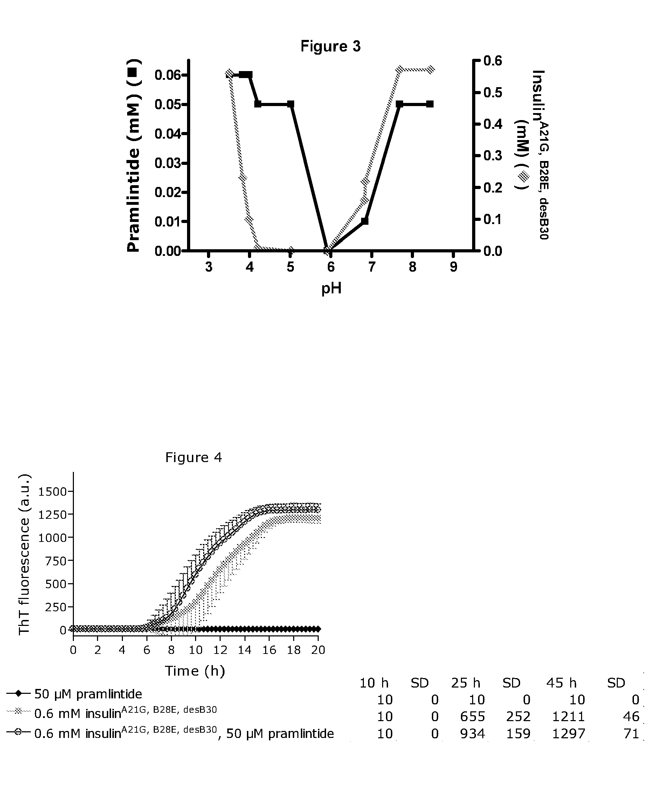

Figure 3 shows the solubility of a mixture of the amylin analogue [25,28, 29]Pro- h-amylin and insulin[A21G, B28E, desB30] versus pH. The insulin analogue has been described in patent application WO2004/080480. The B28E mutation renders the insulin monomeric and hence useful as a meal-related insulin. All samples contained 0.2 mM Zn-acetate, 16 mM m-cresol, 16 mM phenol. The concentration of [25 ,28, 29]Pro- h-amylin in solution versus pH was plotted with black line and symbols and using the left $\gamma$-axis; the concentration of the insulin analogue in solution versus pH was plotted using light grey line and symbols and using the right y-axis. [25, 28, 29]Pro- h-amylin co-precipitated with the insulin analogue in its precipitation zone. At pH below 3.5 and above pH 7.7 substantial amounts of both peptides were soluble in this particular mix of analogues. This enables coformulation of therapeutically relevant doses of both insulin[A21G, B28E, desB30] and [25,28,29]Pro- h-amylin at acidic pH, e.g. pH 3.5.

**Example 4**

The physical stability of a mixture containing insulin[A21G, B28E, desB30] and [25,28,29]Pro- h-amylin was assessed by the use of a ThT fibrillation assay. This is shown in Figure 4. All three formulations contained 174 mM glycerol, 16 mM phenol, 16 mM m-cresol, 30 mM sodium acetate; and were adjusted to pH 3.5. Under these conditions [25,28,29]Pro-h-amylin was inert towards fibrillation throughout the assay time. The insulin analogue alone and a mixture of the insulin analogue and [25,28,29]Pro- h-amylin initiated fibrillation with identical lag times (approx. 6 hours). This indicated that there were no mutual destabilisation since the mixture was just as physical stable as the least stable component (the insulin analogue). This enables a stable mix formulation of the two peptides at acidic pH, e.g. pH 3.5.

**Example 5**

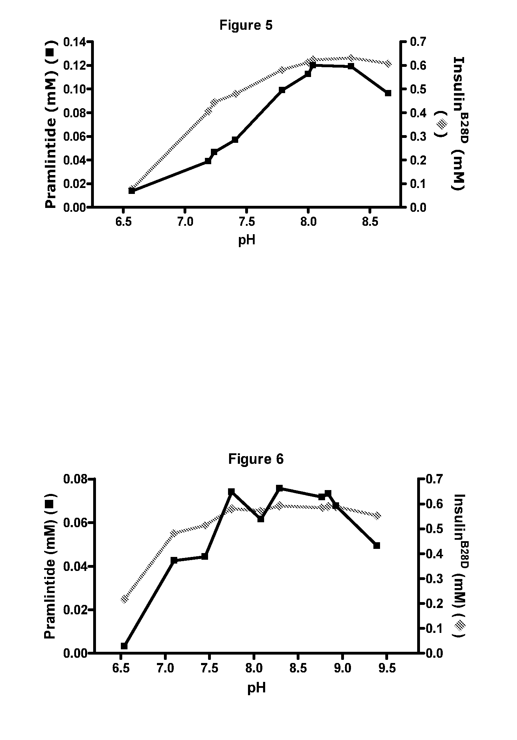

Figure 5 shows the solubility of a mixture of 0.6 mM insulin[B28D], 0.2 mM Zn(Ac)$_2$, 0.15 mM[25, 28, 29]Pro- h-amylin versus pH. The concentration of [25, 28, 29]Pro- h-amylin in solution versus pH was plotted with a black line and symbols and using the left y-axis; the concentration of the insulin analogue in solution versus pH was plotted using a light grey line and symbols and using the right y-axis. Despite co-precipitation at physiological pH, it was possible to have significant amounts of both [25,28,29]Pro- h-amylin and insulin[B28D] in solution at slightly higher pH. This enables simultaneous delivery of both insulin[B28D] and [25, 28, 29]Pro- h-amylin in clinical relevant doses formulated at pH slightly higher than physiological pH, e.g. pH 8.0.

**Example 6**

Figure 6 shows the solubility of a mixture of 0.6mM Insulin$^{B28D}$, 0.2mM Zn(AC)$_2$, 0.1 mM $^{25, 28, 29}$Pro- h-amylin, 16mM phenol, 16mM m-cresol, 3000 ppm Poloxamer-188. The concentration of $^{25, 28, 29}$Pro- h-amylin in solution versus pH was plotted with a black line and symbols and using the left y-axis; the concentration of the insulin analogue in solution versus pH was plotted using a light grey line and symbols and using the right y-axis. The solubility of both peptides increased when pH increased from neutral pH to pH 8.0. This enables simultaneous delivery of both insulin$^{B28D}$ and $^{25, 28, 29}$Pro- h-amylin in clinical relevant doses formulated at pH slightly higher than physiological pH, e.g. pH 8.0. The presence of the surfactant Poloxamer-188 may increase the physical stability of such a coformulation.

**Example 7**

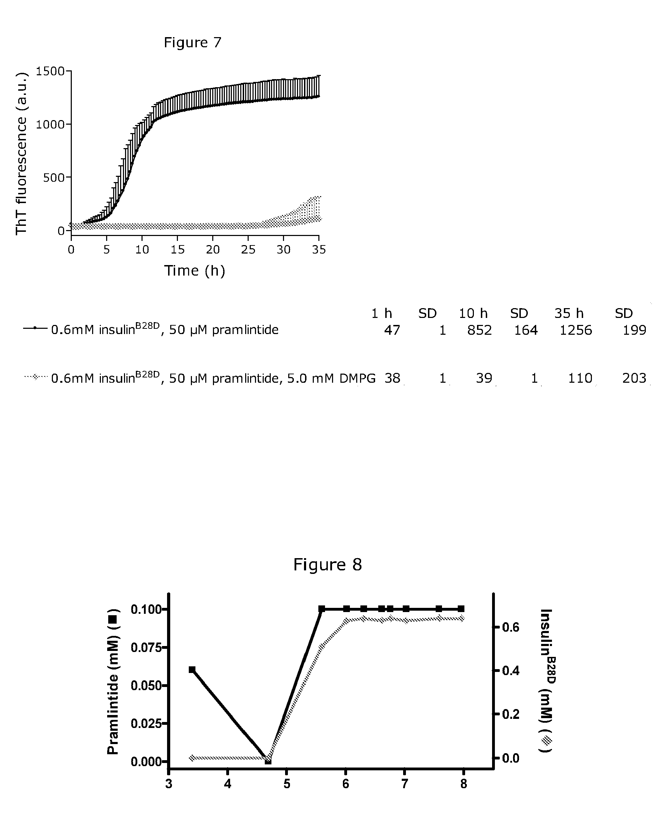

In this example the effect of a phospholipid on the physical stability of a mixture containing both insulin$^{B28D}$ and $^{25, 28, 29}$Pro- h-amylin was examined. This is shown in Figure 7. Both formulations contained 0.6 mM insulin$^{B28D}$, 50 μM $^{25, 28, 29}$Pro- h-amylin, 0.3 mM Zn(Ac)$_2$, 174 mM glycerol, 30 mM phenol, 8 mM glycylglycine pH 7.4. The sample without added surfactant exhibited a significant ThT fluorescence signal after a lag time of approx. 4 hours indicating that fibrillation had initiated. The addition of 1,2-Dimyristoyl-sn-glycero-3-phospho-rac-(1-glycerol) (DMPG), CAS no. 67232-80-8, to 5 mM significantly increased this lagtime to more than 25 hours. This indicates that coformulation of insulin$^{B28D}$ and $^{25, 28, 29}$Pro- h-amylin in the presence of an anionic lipid micelle may increase the physical stability of the formulation.

**Example 8**

The addition of the DMPG phospholipid also increased the solubility of both insulin$^{B28D}$ and $^{25, 28, 29}$Pro- h-amylin. Figure 8 shows the solubility of a mixture of 0.6 mM insulin$^{B28D}$, 0.2 mM Zn(Ac)$_2$, 100 μM $^{25, 28, 29}$Pro- h-amylin 30 mM phenol, and 3 mM DMPG. The concentration of $^{25, 28, 29}$Pro- h-amylin in solution versus pH was plotted with a black line and symbols and using the left y-axis; the concentration of the insulin analogue in solution versus pH was plotted using a light grey line and symbols and using the right y-axis. The effect of DMPG was very pronounced. Full solubility of both insulin$^{B28D}$ and $^{25, 28, 29}$Pro- h-amylin was observed already above pH 6. This enables formulation and subcutaneous injection of therapeutically relevant doses in an acceptable injection volume and at physiological pH. Furthermore, the mix formulation of insulin$^{B28D}$ and $^{25, 28, 29}$Pro- h-amylin with DMPG is expected to be stable against fibrillation as illustrated by Examples 7 and 9.

**Example 9**

The physical stability of mixtures containing insulin$^{B28D}$, $^{25, 28, 29}$Pro- h-amylin, and DMPG was assessed using a ThT fibrillation assay. This is shown in Figure 9. Both formulation 9A and 9B contained 0.6 mM insulin$^{B28D}$, 0.3 mM Zn(Ac)$_2$, 100 μM $^{25, 28, 29}$Pro- h-amylin, 174 mM glycerol, 30 mM phenol, 8 mM glycylglycine pH 7.4. Formulation 9A further contained 1.0 mM DMPG, and Formulation 9B further contained 3.0 mM DMPG. Formulation 9A (containing 1.0 mM DMPG) had a lag time of approximately 2.5 hours, whereas Formulation 9B (containing 3.0 mM DMPG) had a lag time of approximately 22 hours before fibrillation initiated. This indicated that DMPG increased the physical stability of the insulin$^{B28D}$ - $^{25, 28, 29}$Pro- h-amylin coformulation, and the lag time before fibrillation occurred was significantly prolonged in the presence of 3.0 mM DMPG.

**Claims**

**1.** A soluble pharmaceutical composition for parenteral administration, which comprises an amylin peptide, and a meal-related insulin peptide, wherein the composition comprises an anionic surfactant.

**2.** The pharmaceutical composition according to claim 1, wherein said anionic surfactant is a glycerophosphoglycerol derivative.

**3.** The pharmaceutical composition according to claim 2, wherein said glycerophosphoglycerol derivative is a dimyristoyl derivative.

**4.** The pharmaceutical composition according to claim 2, wherein said said glycerophosphoglycerol derivative is 1,2-Dimyristoyl-sn-glycero-3-phospho-rac-(1-glycerol) (DMPG).

**5.** The pharmaceutical composition according to any of the claims 1-4, wherein the surfactant is added in a concentration between 0.1 mM and 10 mM.

**6.** A pharmaceutical composition according to any of claims 1-5 for use as a medicament in treatment of hyperglycemia.

**7.** A pharmaceutical composition according to claim 6, comprising parenteral administration of an effective amount of the pharmaceutical composition according to any one of the claims 1-6.

**8.** A pharmaceutical composition according to claim 1, wherein said meal-related insulin peptide is human insulin.
9. A pharmaceutical composition according to claim 1, wherein said meal-related insulin peptide is a human insulin analog.

**10.** The pharmaceutical composition according to claim 1, wherein said meal-related insulin peptide is a derivative of a human insulin analog.

**11.** The pharmaceutical composition according to claim 1, wherein said amylin peptide is amylin, an amylin analog or an amylin agonist.

**12.** The pharmaceutical according to claim 1, wherein said amylin peptide is human amylin.

**13.** The pharmaceutical composition according to claim 1, wherein said amylin peptide is $^{25,28,29}$Pro- h-amylin.

**14.** The pharmaceutical composition according to claim 1, wherein said meal-related insulin peptide is human insulin and said amylin peptide is $^{25,28,29}$Pro- h-amylin.15. An insulin peptide and an amylin peptide according to claim 1 for use as a medicament for the treatment of binge eating or bulimia.

## Figure 1

## Figure 2

| | 4 h | SD | 8 h | SD | 12 h | SD |
|---|---|---|---|---|---|---|
| 50 μM pramlintide | 10 | 0 | 10 | 1 | 10 | 0 |
| 0.6 mM insulin$^{A21G, B28D, desB30}$ | 14 | 8 | 1081 | 655 | 1392 | 455 |
| 0.6 mM insulin$^{A21G, B28D, desB30}$, 50μM pramlintide | 14 | 9 | 1106 | 562 | 1493 | 119 |

**Figure 3**

**Figure 4**

| | 10 h | SD | 25 h | SD | 45 h | SD |
|---|---|---|---|---|---|---|
| 50 μM pramlintide | 10 | 0 | 10 | 0 | 10 | 0 |
| 0.6 mM insulin$^{A21G, B28E, desB30}$ | 10 | 0 | 655 | 252 | 1211 | 46 |
| 0.6 mM insulin$^{A21G, B28E, desB30}$, 50 μM pramlintide | 10 | 0 | 934 | 159 | 1297 | 71 |

**Figure 5**

**Figure 6**

## Figure 7

|  | 1 h | SD | 10 h | SD | 35 h | SD |
|---|---|---|---|---|---|---|
| ● 0.6mM insulin$^{B28D}$, 50 µM pramlintide | 47 | 1 | 852 | 164 | 1256 | 199 |
| ···◈··· 0.6mM insulin$^{B28D}$, 50 µM pramlintide, 5.0 mM DMPG | 38 | 1 | 39 | 1 | 110 | 203 |

## Figure 8

Figure 9

| | 2 h | SD | 20 h | SD | 40 h | SD |
|---|---|---|---|---|---|---|
| ~~~ Formulation 9A (+ 1.0 mM DMPG) | 8 | 0 | 900 | 51 | 911 | 40 |
| —o— Formulation 9B (+ 3.0 mM DMPG) | 7 | 0 | 8 | 1 | 388 | 395 |

Figure 10

**EUROPEAN SEARCH REPORT**

Application Number

EP 10 16 6765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 99/34764 A (AMYLIN PHARMACEUTICALS INC [US]) 15 July 1999 (1999-07-15) * see claims 1-4, examples 2-4, page 78 lines 15-20, page 41 line 10 to page 43 line 5 * | 1-15 | INV. A61K38/28 A61K38/22 A61K47/10 A61P3/10 |
| Y | WO 03/101395 A (AMYLIN PHARMACEUTICALS INC [US]; L ITALIEN JAMES J [US]; STETSKO GREGG) 11 December 2003 (2003-12-11) * see claims 1-17 and 48, paragraph [0045], pages 31-34 and tables C to I * | 1-15 | |
| A | WO 01/54742 A (AEROPHARM TECHNOLOGY INC [US]) 2 August 2001 (2001-08-02) * see claims 2-4 and 10-12 * | 1-15 | |
| A,D | WO 2004/037168 A (AMYLIN PHARMACEUTICALS INC [US]; GEDULIN BRONISLAVA [US]; YOUNG ANDREW) 6 May 2004 (2004-05-06) * see claims 1, 9-11 and pages 15-16 * | 1-15 | |
| Y | WO 01/00223 A (MINIMED INC [US]) 4 January 2001 (2001-01-04) * see claims 1-13 * | 1-15 | TECHNICAL FIELDS SEARCHED (IPC) A61K A61P |

The present search report has been drawn up for all claims

| Place of search | Date of completion of the search | Examiner |
|---|---|---|
| Munich | 31 August 2010 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**

**ON EUROPEAN PATENT APPLICATION NO.**                    EP 10 16 6765

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2010

| Patent document cited in search report | | Publication date | Patent family member(s) | | Publication date |
|---|---|---|---|---|---|
| WO 9934764 | A | 15-07-1999 | ZA | 9800221 A | 25-11-1998 |
| WO 03101395 | A | 11-12-2003 | AU | 2003238862 A1 | 19-12-2003 |
| | | | CA | 2487585 A1 | 11-12-2003 |
| | | | EP | 1551436 A2 | 13-07-2005 |
| | | | JP | 2005537232 T | 08-12-2005 |
| | | | NZ | 536813 A | 31-05-2007 |
| WO 0154742 | A | 02-08-2001 | AU | 783134 B2 | 29-09-2005 |
| | | | AU | 2752901 A | 07-08-2001 |
| | | | CA | 2396648 A1 | 02-08-2001 |
| | | | CN | 1418113 A | 14-05-2003 |
| | | | EP | 1296723 A1 | 02-04-2003 |
| | | | JP | 2003524643 T | 19-08-2003 |
| | | | MX | PA02007189 A | 09-12-2002 |
| | | | US | 6540982 B1 | 01-04-2003 |
| WO 2004037168 | A | 06-05-2004 | AU | 2003278929 A1 | 13-05-2004 |
| WO 0100223 | A | 04-01-2001 | AU | 5760900 A | 31-01-2001 |
| | | | CA | 2369839 A1 | 04-01-2001 |
| | | | EP | 1196189 A2 | 17-04-2002 |
| | | | JP | 2003503356 T | 28-01-2003 |
| | | | US | 2006074013 A1 | 06-04-2006 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0792290 A
- EP 0214826 A
- EP 0705275 A
- US 5504188 A
- EP 0368187 A
- US 5750497 A
- US 6011007 A
- EP 375437 A
- EP 383472 A
- WO 2004080480 A
- US 5124314 A

- US 5234906 A
- WO 2004037168 A
- EP 289287 A
- US 5686411 A
- US 6114304 A
- US 6410511 B
- WO 9808871 A
- WO 9943706 A
- US 5424286 A
- WO 0009666 A

**Non-patent literature cited in the description**

- **A. Young.** Amylin: Physiology and Pharmacology. Academic Press, 2005
- Surfactants and Polymers in Aqueous Solution
- **Yan et al.** *PNAS,* 2006, vol. 103 (7), 2046-2051
- **Naiki et al.** *Anal. Biochem.,* 1989, vol. 177, 244-249

- **LeVine.** *Methods. Enzymol.,* 1999, vol. 309, 274-284
- **Nielsen et al.** *Biochemistry,* 2001, vol. 40, 6036-6046
- **Poyner DR et al.** *Pharmacological Reviews,* 2002, vol. 54 (2), 233-246

SEQUENCE LISTING

```
<110>  Novo Nordisk A/S

<120>  Mixtures of Amylin and Insulin

<130>  7376.215-EP

<160>  1

<170>  PatentIn version 3.3

<210>  1
<211>  37
<212>  PRT
<213>  Homo sapiens


<220>
<221>  DISULFIDE
<222>  (2)..(7)

<220>
<221>  Terminal amide
<222>  (37)


<400>  1

Lys Cys Asn Thr Ala Thr Cys Ala Thr Gln Arg Leu Ala Asn Phe Leu
1               5                   10                  15


Val His Ser Ser Asn Asn Phe Gly Ala Ile Leu Ser Ser Thr Asn Val
            20                  25                  30


Gly Ser Asn Thr Tyr
            35
```